# EUROPEAN PATENT APPLICATION

(11) **EP 3 446 673 A1**
(43) Date of publication of application: **27.02.2019**
(21) Application number: 17785872.7
(22) Date of filing: 12.04.2017
(51) Int. Cl.: A61J 3/00, G06K 19/06, G06T 7/00

(54) **IMAGE PROCESSING PROGRAM AND IMAGE PROCESSING DEVICE**

(30) Priority: 19.04.2016 JP 2016083485; 26.05.2016 JP 2016105421; 30.03.2017 JP 2017066820
(71) Applicant: Yuyama Mfg. Co., Ltd., Toyonaka-shi Osaka 561-0841 (JP)
(72) Inventor: SUGIMOTO, Tomohiro, Toyonaka-shi Osaka 561-0841 (JP); ITO, Koji, Toyonaka-shi Osaka 561-0841 (JP); MATSUNAGA, Maki, Toyonaka-shi Osaka 561-0841 (JP)
(74) Representative: AOMB Polska Sp. z.o.o.
(86) International application number: PCT/JP2017/014944
(87) International publication number: WO 2017/183533

(57) **Abstract**

An image processing program 600 makes a computer execute the steps of: displaying an image photographing a region including plurality of medicines (710-1)-(710-3), (720) on a display device, an displaying the images of the plurality of medicines (710-1)-(710-3), (720) on the display device with sorting depending on a similarity degree between an image of a representative medicine (710-2) selected from the plurality of medicines (710-1)-(710-3), (720) displayed on the display device and each of the images of the plurality of medicines (710-1)-(710-3), (720).

## Description

### FIELD OF INVENTION

The present invention relates to an image processing program and an image processing apparatus.

### BACKGROUND ART

Currently, there are many cases that one patient has medical examinations at a plurality of medical institutions. In this case, a physician must understand medicines prescribed to the patient at the other medical institution. Thus, the physician often requests a patient to bring medicines prescribed at the other medical institution. In such cases, when an identification code of a medicine is printed on a bag for packaging the medicine, the medicine prescribed could be understood; however, there are many cases in which the code is not printed or only the medicine is brought nakedly. In such cases, the medical institute must identify the medicine prescribed only by using an appearance of the medicine as a clue. Such work is called as judgement work and usually as a job for a pharmacist or a technician Practically, pharmacists perform the judgement of unknown medicines manually by using sizes, shapes, and/or colors as clues. Unfortunately, the judgement work is hard and requires large time consumption and imposes large loads.

Furthermore, in a medical institute, there are cases that the medicines prescribed once to hospitalized patients are returned without taken. For safe reuse of the returned medicines, there are cases that the returned medicines are subjected to the judgement; however the judgement work requires large time consumption to exert large loads to pharmacists. In order to reduce such loads, a medicine judgement apparatus has been proposed.

### PRIOR ART LITERATURE

### PATENT LITERATURE

Patent Literature 1: Japan Patent Publication (Laid-open) No. Heisei 9-16681
Patent Literature 2: Japan Patent Publication (Laid-open) No. Heisei 5-245186

### SUMMARY OF INVENTION

### PROBLEM TO BE SOLVED BY INVENTION

However, such apparatuses are still in topics in literatures described levels and do not come into practical use. In order to accomplish as a product which may be used practically, many developments will be necessary in various aspects of a medicine judgement system including an apparatus, and software, and the like.

For example, in a medicine judgement apparatus, various images for judging medicines are presented on a display device to workers taking part in the judgement and it is important to present on the display device the images which are able to improve accuracy of the judgement.

An object of one embodiment of the present invention is to contribute improvement of judgement accuracy for medicines.

### MEANS FOR SOLVING PROBLEM

A program of one embodiment of the present invention makes a computer execute the steps of: displaying on a display device an image taking a picture of a region including a plurality of medicines, and displaying the images of the plurality of medicines on the display device with sorting depending on a similarity degree between an image of a representative medicine selected from the plurality of medicines displayed on the display device and each of the images of the plurality of medicines.

An image processing apparatus of one embodiment of the present invention comprises a receiver part for receiving an image taking a picture of a region including plurality of medicines by a medicine photographing apparatus and an image processing part for executing the steps of; displaying an image photographing a region including plurality of medicines on a display device; and displaying the images of the plurality of medicines on the display device with sorting depending on a similarity degree between an image of a representative medicine selected from the plurality of medicines displayed on the display device and each of the images of the plurality of medicines.

### ADVANTAGE OF INVENTION

According to one embodiment of the present invention, contribution to improvement of judgment accuracy for medicines may be provided.

### BRIEF EXPLANATION OF DRAWINGS

[Fig. 1] Fig. 1 depicts a schematic illustration which depicts generally a medicine judgement system.
[Fig. 2] Fig. 2 illustrates a perspective view of a medicine photographing device.
[Fig. 3] Fig. 3 depicts a state that the cover of the medicine photographing device is opened.
[Fig. 4] Fig. 4 illustrate a construction of the inside of the medicine photographing device.
[Fig. 5] Fig. 5 illustrates a front view of the medicine photographing device show in Fig. 4.
[Fig. 6] Fig. 6 illustrates an image of a screen at an early stage after a medicine judgement software is started.
[Fig. 7] Fig. 7 illustrates an image of a screen at a first step of a returned tablet image judgement.
[Fig. 8] Fig. 8 illustrates an image of a screen at a second step of a returned tablet image judgement started from Fig. 7.
[Fig. 9] Fig. 9 illustrates an image of a screen at a third step of a returned tablet image judgement started from Fig. 7.
[Fig. 10] Fig. 10 illustrates an image of a screen at a fourth step of a returned tablet image judgement started from Fig. 7.
[Fig. 11] Fig. 11 illustrates an image of a screen at a fifth step of a returned tablet image judgement started from Fig. 7.
[Fig. 12] Fig. 12 illustrates a drawing for explaining sorting of images of a plurality of medicines and shows a state that a plurality of medicines are placed.
[Fig. 13 Fig. 13 illustrates a drawing of a display to a third part of a plurality of medicines by a general medicine judgement program.
[Fig. 14] Fig. 14 illustrates a drawing of a display to a third part of a plurality of medicines by a medicine judgement program according to the present invention.
[Fig. 15] Fig. 15 illustrates a drawing of one example of a printed returned medicine judgement result.
[Fig. 16] Fig. 16 is a flowchart executed by an image processing program.
[Fig. 17] Fig. 17 is a schematic drawing illustrating an outline of the medicine folding system of the present invention.
[Fig. 18] Fig. 18 is a drawing of one example for an inquiry screen of a filling history of the medicine filled in the folding machine.
[Fig. 19] Fig. 19 is a drawing of one example for an inquiry screen of a filling history of the medicine filled in the folding machine.
[Fig. 20] Fig. 20 illustrates a drawing of one example of a printed returned medicine judgement result.
[Fig. 21] Fig. 21 illustrates one example of an environment setting window for setting whether or not the total number of the judged medicines and the total medicine price are printed.
[Fig. 22] Fig. 22 illustrates one example of a picture image in the judgement of the brought-in medicines.
Fig. 23 is a flowchart of the accumulation work for working time and costs required for judgement of the medicine.

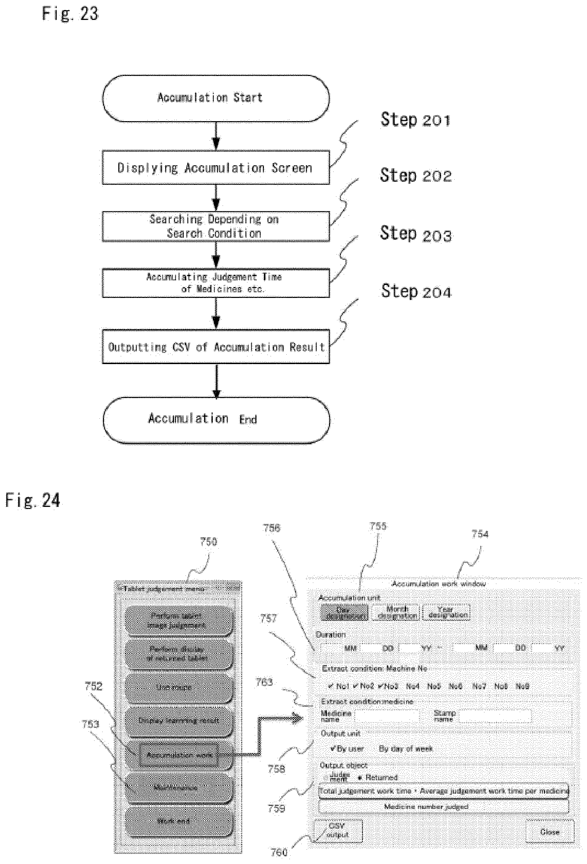

[Fig. 23] Fig. 23 is a flowchart of the accumulation work for working time and costs required for judgement of the medicine.
[Fig. 24] Fig. 24 is one example of screen for accumulation about the working time and costs required for the judgement of the medicine.

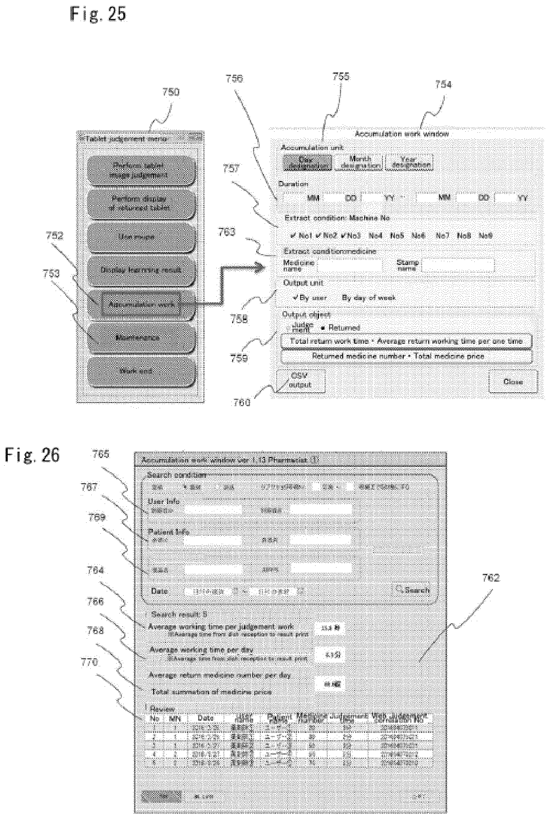

[Fig. 25] Fig. 25 is one example of screen for accumulation about the working time and costs required for the judgement of the medicine.
[Fig. 26] Fig. 26 is a drawing showing one example of the accumulation result screen for the judgement time and the like for judging the medicine.

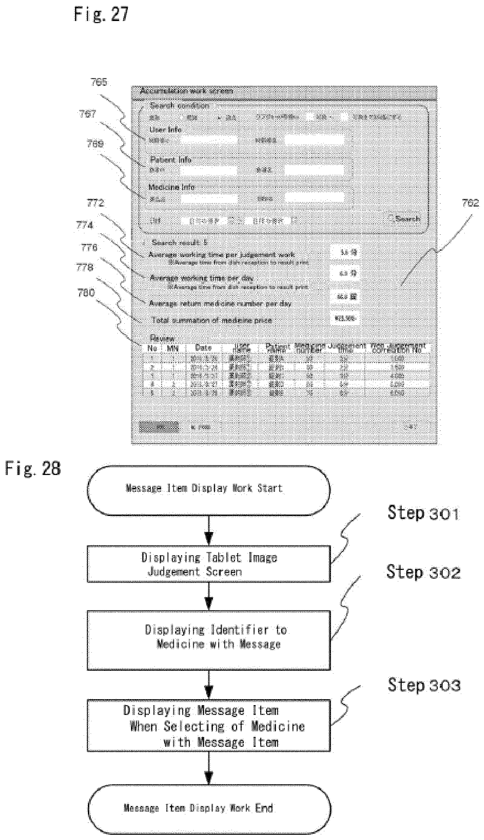

[Fig. 27] Fig. 27 is a drawing showing one example of the accumulation result screen for the judgement time and the like for judging the medicine.
[Fig. 28] Fig. 28 is a flowchart of the message display work.

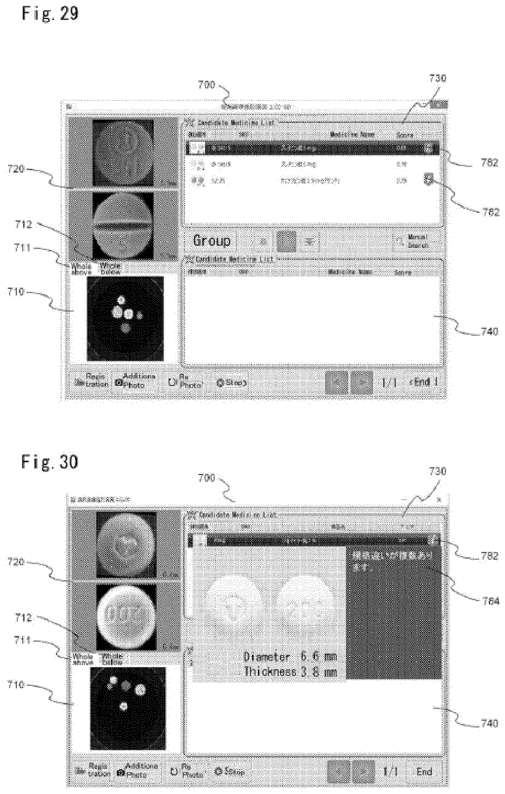

[Fig. 29] Fig. 29 is drawing of one example of a condition displaying a message item about medicines.
[Fig. 30] Fig. 30 is a drawing of one example of a condition displaying a message item about a medicine judgement.

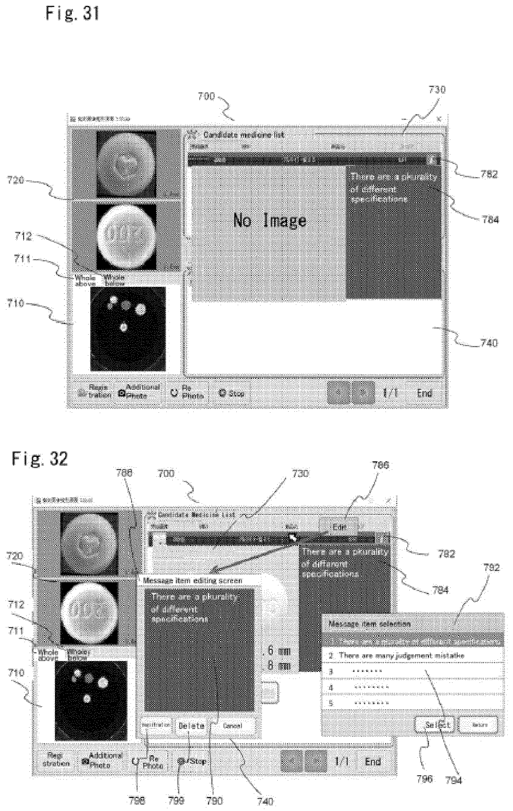

[Fig. 31] Fig. 31 is a drawing of one example of a condition displaying presence or absence of a message item about a medicine judgement.
[Fig. 32] Fig. 32 a drawing of one example of a screen for editing a message item about a medicine judgement.

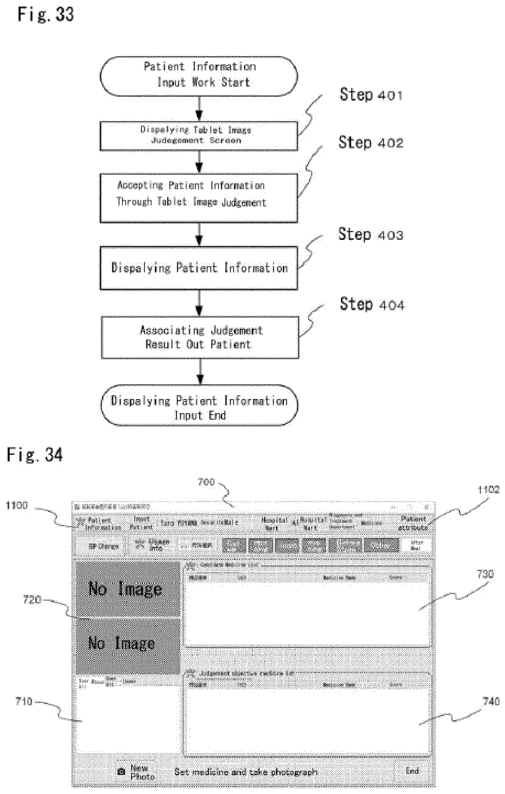

[Fig. 33] Fig. 33 shown a flowchart of a patient information input work.
[Fig. 34] Fig. 34 is a drawing of one example of a state displaying information about a patient brought medicines in a screen for a medicine judgement.

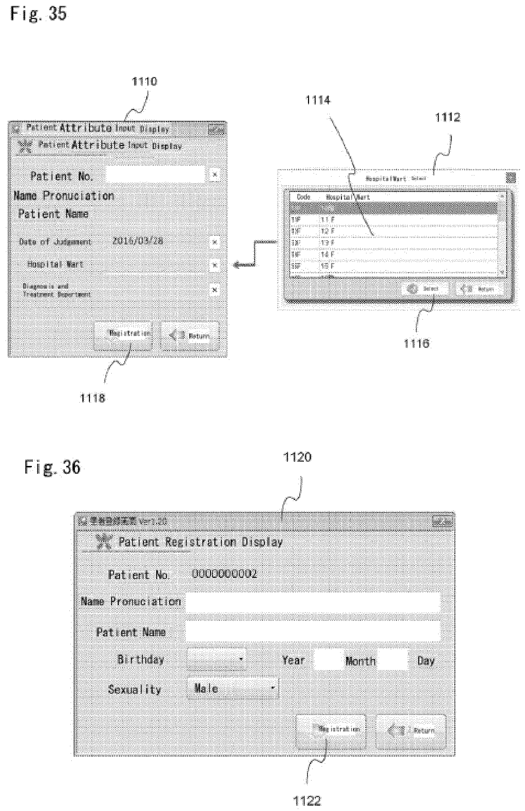

[Fig. 35] Fig. 34 is a drawing of one example of a screen for inputting an attribute of a patient brought medicines.
[Fig. 36] Fig. 36 is a drawing of one example of a screen for inputting an attribute of a patient brought medicines.

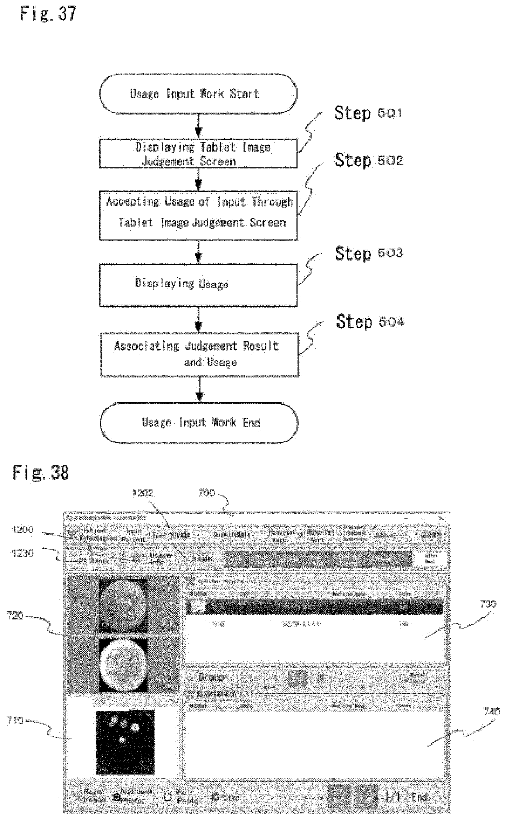

[Fig. 37] Fig, 37 is a flowchart of a usage input work.
[Fig. 38] Fig. 38 is a drawing of one example of a judgement screen for judging medicines brought by a patient.

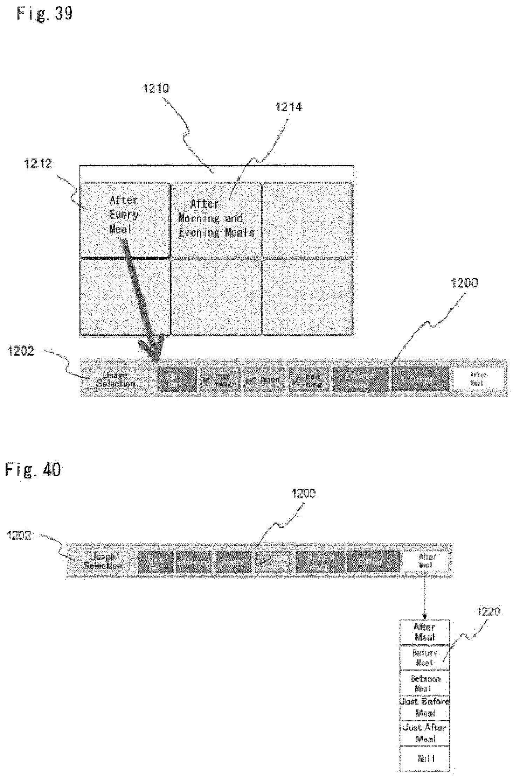

[Fig. 39] Fig. 39 is a drawing of one example of the screen for inputting the usage of the medicine that the patient brought through the judgement screen for judging the medicine that the patient brought.
[Fig. 40] Fig. 40 is a drawing of one example of the screen for inputting the usage of the medicine that the patient brought through the judgement screen for judging the medicine that the patient brought.

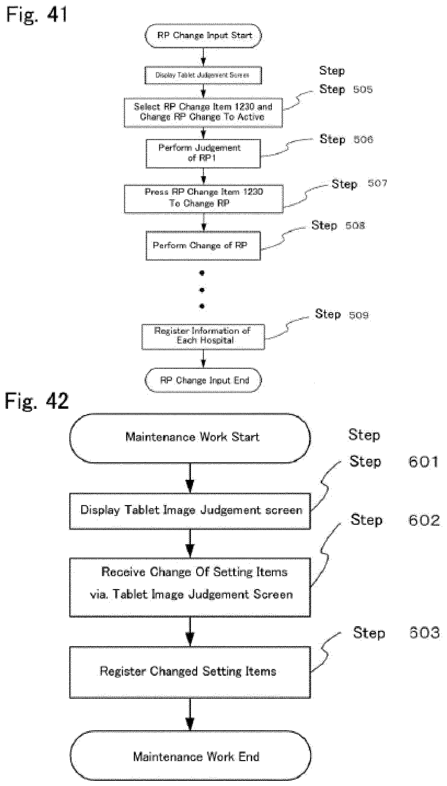

[Fig. 41] Fig. 41 is a flowchart of RP switching work.
[Fig. 42] Fig. 42 is a flowchart of a maintenance work.

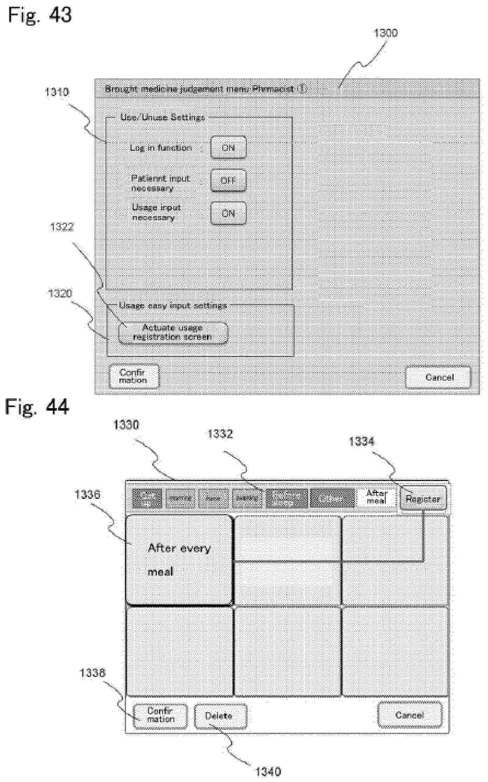

[Fig. 43] Fig. 43 is a drawing of one example for changing the settings about the medicine judgement.
[Fig. 44] Fig. 44 is a drawing of one example of the screen for registering the usage of the medicines.

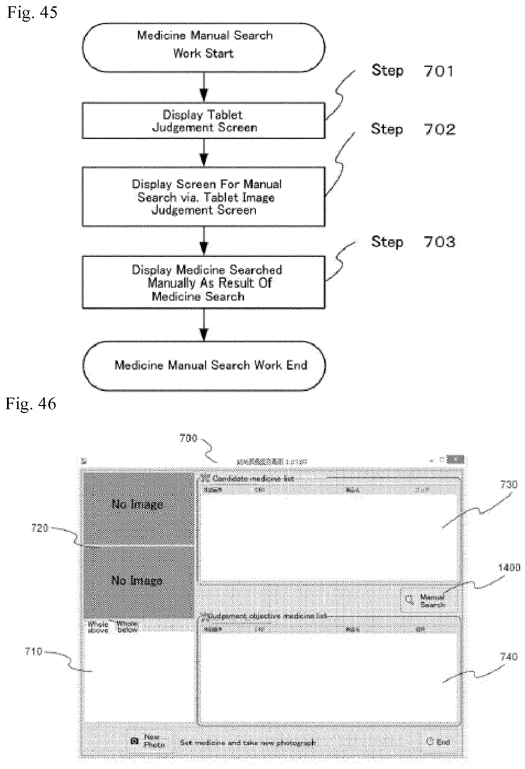

[Fig. 45] Fig. 45 is a flowchart of the manual search work of the judgement medicines.
[Fig. 46] Fig. 46 is a drawing of one example of a screen for the manual search through the judgement screen for judging the medicines.

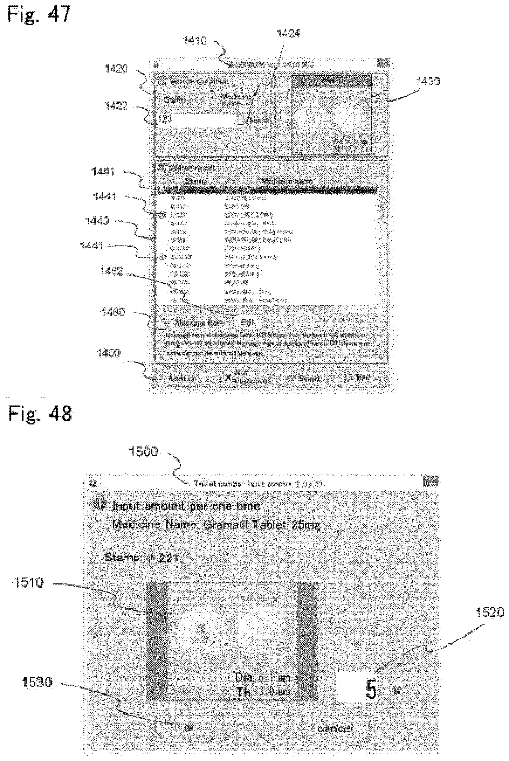

[Fig. 47] Fig. 47 is a drawing of one example of the screen for manual search of the medicine.
[Fig. 48] Fig. 48 is a drawing of one example of the screen for manual search of the medicine.

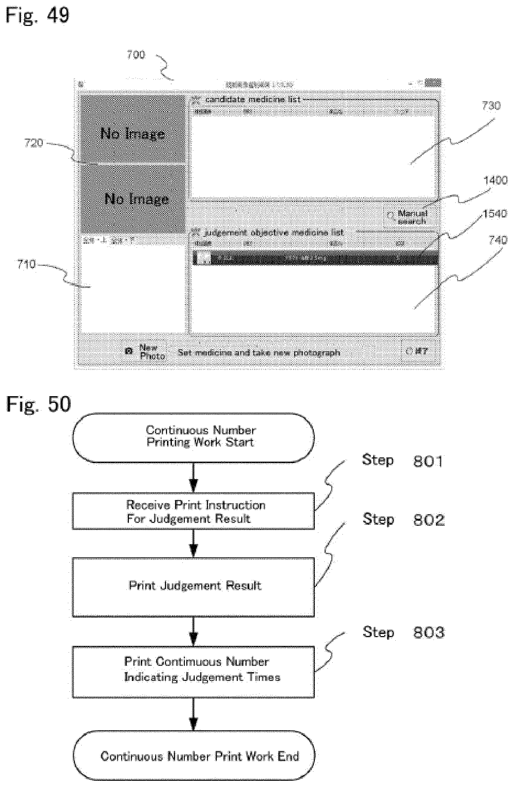

[Fig. 49] Fig. 49 is a drawing of one example for the condition in that the medicine manually searched is displayed on the judgement screen as the judgement result.
[Fig. 50] Fig. 50 is a flowchart of continuous number printing work.

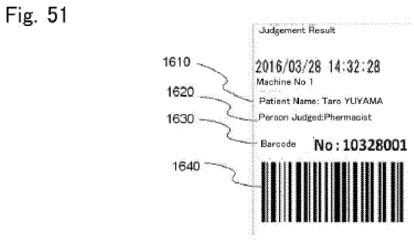

[Fig. 51] Fig. 51 is a drawing of one example of the printed medicine judgement result.

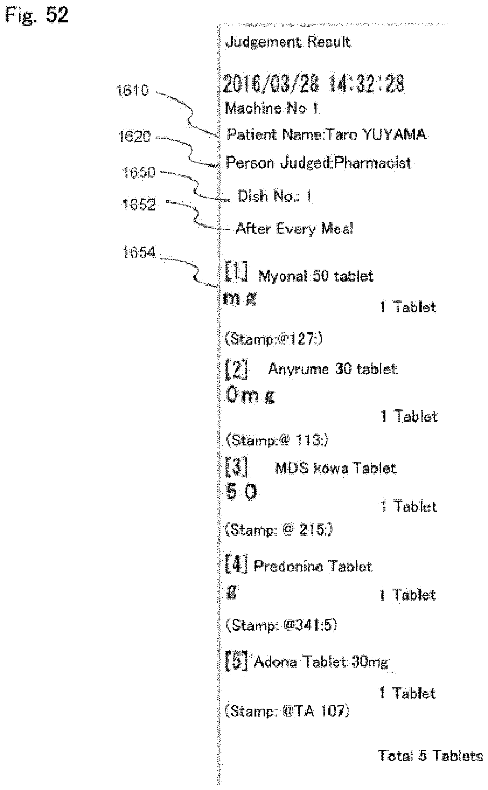

[Fig. 52] Fig. 52 is a drawing of one example of the printed medicine judgement result.

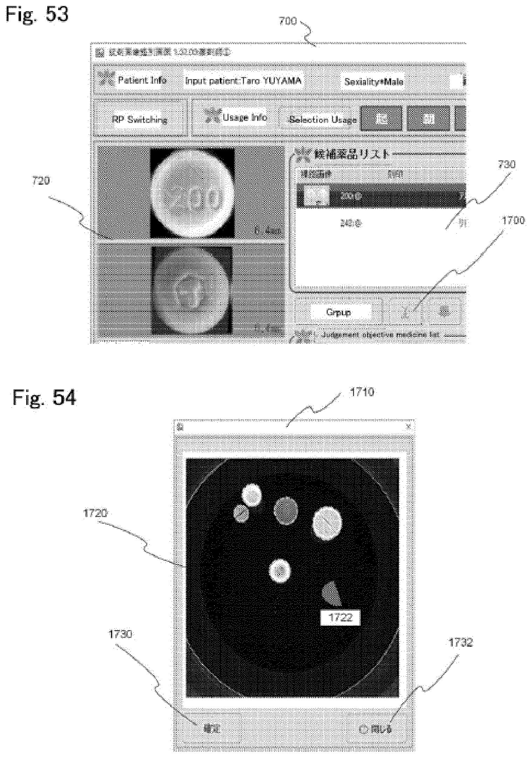

[Fig. 53] Fig. 53 is a drawing for explaining the region editing function
[Fig. 54] Fig. 54 is a drawing of one example of the region editing screen.

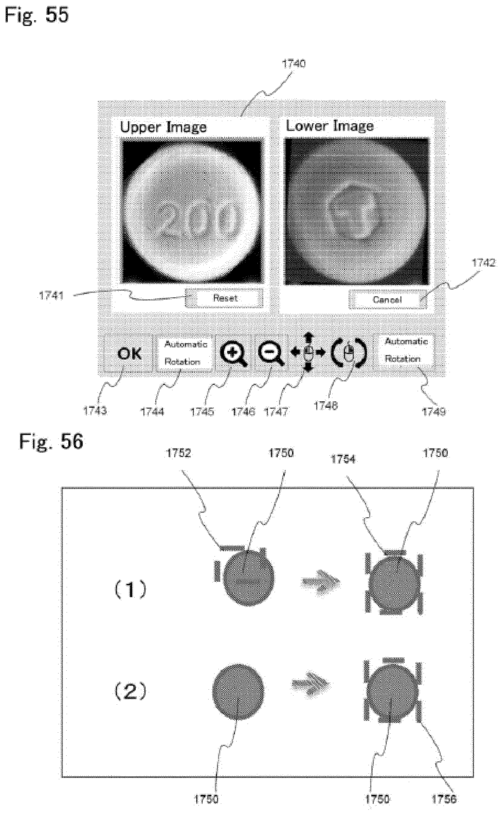

[Fig. 55] Fig. 55 is a drawing of one example of the cut-out region editing screen.
[Fig. 56] Fig. 56 is a schematic illustration of one example of the region editing for the medicine.

### MODE FOR PRACTICING INVENTION

Now, embodiments of the present invention will be explained in detail with referring to drawings. In each drawing, the same reference signs are provided with construction elements which exhibits the same or similar function and duplicated explanation will be omitted.

### Section 1 Medicine Judgement System

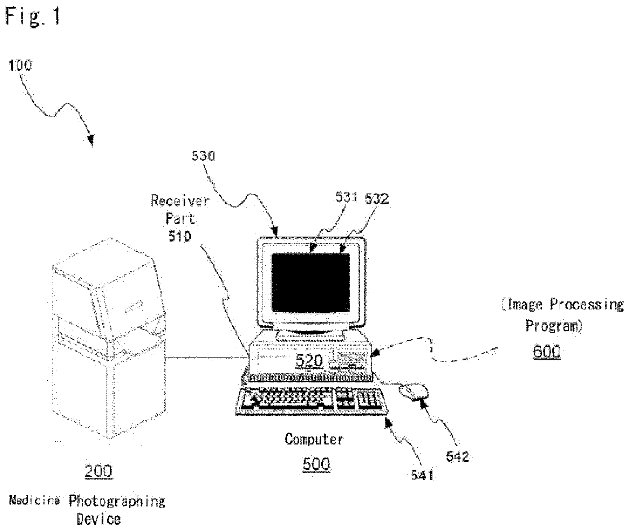

Fig. 1 depicts a schematic illustration which depicts generally a medicine judgement system. As described in the figure, the medicine judgement system comprises a medicine photographing device 200 and a computer 500. In this medicine judgement system 100, the medicine photographing device 200 takes a photograph of the medicines and the computer 500 performs the judgement of the medicine depending on obtained image data of the medicine.

The computer 500 may comprise a receiver part 510, a main body 520 which encloses a CPU (image processing part), a storage part, a recording device, and a display device 530 which functions as an output device as well as a keyboard 541 and a mouse 542 which function as an input device. In addition, the display device 530 may comprise a display screen 531 and a touch screen 532 and a user can perform input to the computer 500 through the touch screen 532. Furthermore, an image processing program 600 may be installed in the computer 500. In the above sense, the computer 500 to which the image processing program (which may be referred as image processing software) 600 is installed, may be referred as an image processing apparatus. The image processing program 600, as for one example, makes the computer 500 to execute various processing about the medicine judgement and may be referred as a medicine judgement program. In this case, the medicine judgement program is installed in the computer 500 and then the computer 500 may also be referred as a medicine judgement apparatus. Now, according to the present embodiment, the computer 500 may be composed of a so-called PC (personal computer) and the image processing program 600 is stored in the storage device; however, the computer 500 may be, for example, a microcomputer embedded into the other apparatus. In this case, the image processing program 600 may be written in a storage device of the other apparatus such as ROM (read only memory), and the like. Besides, the CPU of the main body 520 may also be referred as an image processing part which executes various image processing depending on the image processing program 600.

When judging the medicine, first a user (for example, a pharmacist etc.) sets to the medicine photographing device 200 the medicines that it wants to judge. Next, with operation of the computer 500 by the user as a trigger, the medicine photographing device 200 takes a photograph of the medicine. The photographed image of the medicine is sent as data to the computer. Then, the computer 500 searches the medicine with referring to a database (not shown) depending on these image data.

In one embodiment, the computer 500 may include the database. For example, when a pharmaceutical integrated database "MD bank" (Trademark) from (Stock Company) YUYAMA MFG., CO., LTD. is installed to the computer 500, the computer 500 may search the medicines by referring to MD bank without accessing to an external database. In the other embodiment, the computer 500 may access to the database through a network. For example, when an administration control supporting system "Pharma Road" (Trademark) from (Stock Company) YUYAMAMFG., CO., LTD. is installed to another computer (server) in the same institute, the computer 500 may search the medicine by accessing to Pharma Road in the server through a LAN (local area network). Furthermore, in the other embodiment, the computer 500 may access to the database through Internet.

Hereinafter, the medicine photographing device 200 will be described in detail. Thereafter, the image processing program 600 will be described in detail.

### Section 2 Medicine Photographing Device

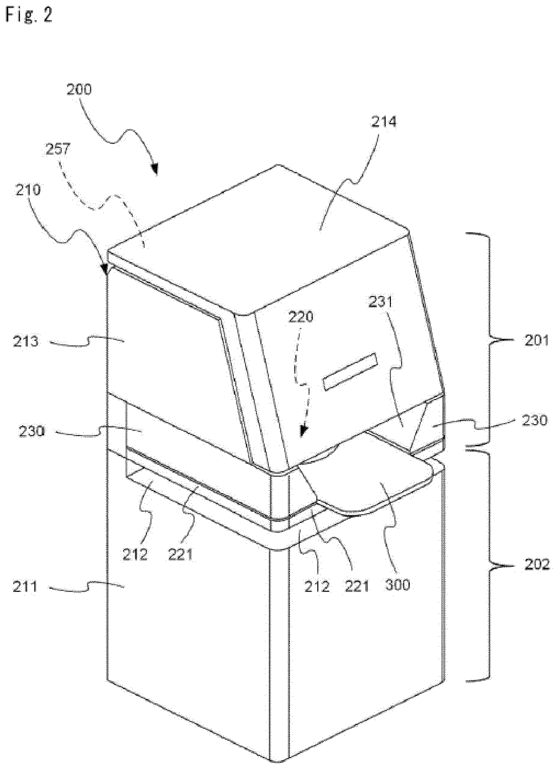

Fig. 2 illustrates a perspective view of an outer appearance of a medicine photographing device 200. In this figure, the inside is hindered by a case 210; at about the center of the inside of the medicine photographing device 200, a loading part 220 for loading the medicine is disposed. More particularly, the medicines are received on a tray 300 shown in Fig. 2 and this tray 300 is set on the loading part 220. Hereinafter, for convenience of explanations, in the medicine photographing device 200, an upper portion than the loading part 220 is referred to an upper part 201 and a lower portion is referred to a lower part 202.

### Section 2.1 Outer Construction of Medicine Photographing Device

As shown in Fig. 2, in the medicine photographing device 200, a lower case 211, a notch part 212, a tray support member 221, a guide member 230, an upper case 213, and a cover 214 can be seen from the outside.

The lower case 211 covers the lower part 202 of the medicine photographing device 200. At an upper face of the lower case 211, a notch part 212 extending horizontally is formed. This notch part 212 is opened toward right, left and front directions of the medicine photographing device 200. Through this notch part 212, the user can insert a powder wrapping paper provided in a longitudinally extending shape into the medicine photographing device 200. Particularly, the powder wrapping paper is spread to the horizontal direction and then is inserted into the notch part 212 from the front side. Then the powder wrapping paper is made to move to a near center part of the medicine photographing device 200. Thereby, without cutting the powder wrapping paper, the medicine packed inside the powder wrapping paper can be photographed with an embedded camera of the medicine photographing device 200. An inside space of the medicine photographing device 200 communicates to the outside space at left and right direction such that the user can insert the wrapping paper into the inside of the medicine photographing device 200 in the case that the powder wrapping paper is rewound even when the powder wrapping paper is longer than a width of the medicine photographing device 200.

At just above the notch part 212, the tray support member 221 is disposed for supporting a member for receiving the medicine. Particularly, on to this tray support member 221, the tray 300 is placed.

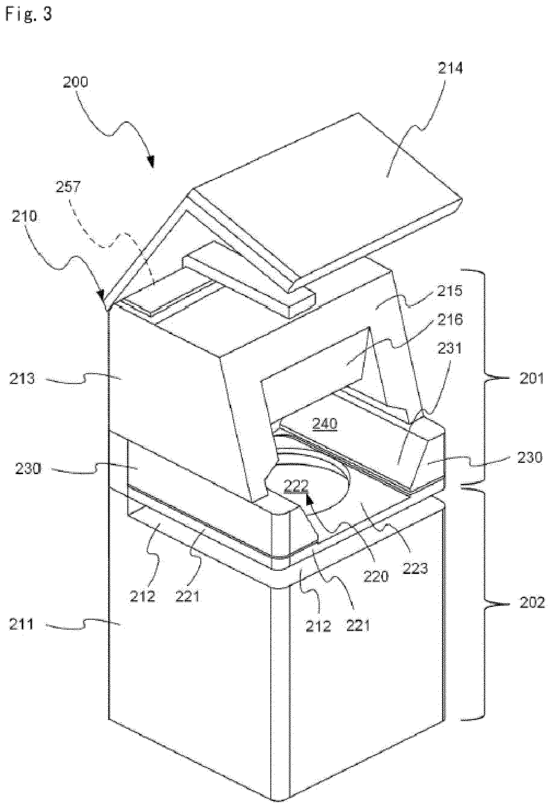

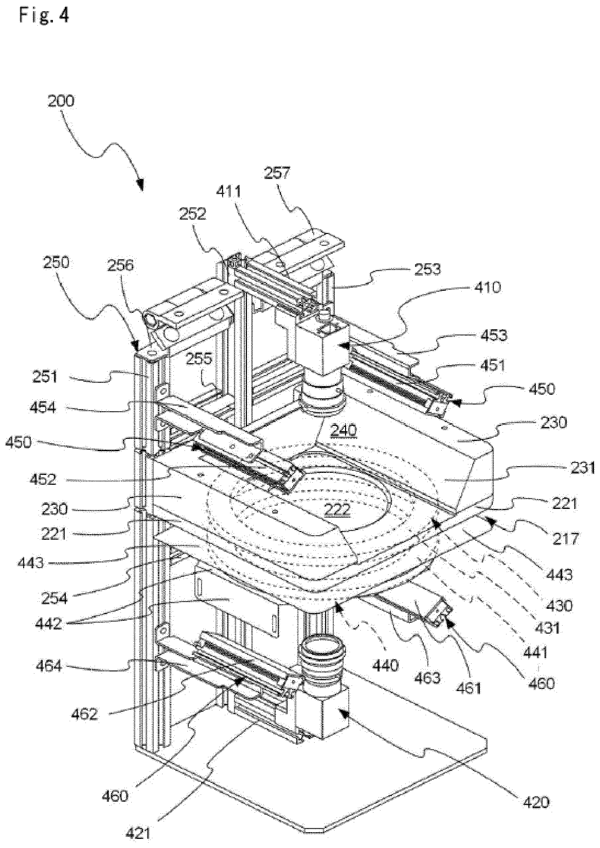

As shown in Fig. 2, the upper face of the medicine photographing device 200 and the upper part 201 of the front are covered with a cover 214. As shown in Fig. 3, the cover 214 may be opened toward the upper direction. As for detail, the cover 214 is rotatably fixed at an back upper end of the upper case 213 by a hinge 257 (refer to Fig. 4) and the cover 214 may be rotatable to upper and lower directions about the hinge 257.

Fig. 3 depicts a state that the cover 214 of the medicine photographing device 200 is opened. As shown in the figure, when the cover 214 is opened, an upper inside space 240, which is located above the upper portion 201 of the inside space in the medicine photographing device 200, is opened toward the front space of the medicine photographing device 200. Then the user can set the tray 300 through the upper inside space 240 to an engagement part 223 of the loading part 220.

As shown in Fig. 3, an outer face at the front side of the upper case 213 is shaped to a near reversed U-shape. The outer face at the front side is slanted, thereby a slanted part 215 is formed. Particularly, the slanted part 215 is inclined such that a distance to a back face of the medicine photographing device 200 becomes small as moving from bottom to top. By this construction, it become easy for the user to insert the tray 300 into the upper inside space 240. There are many cases in that the user moves the tray 300 from diagonally upward to diagonally bottom directions when inserting the tray 300 into the upper inside space 240. In such movement, since the slanted part 215 is inclined, it goes inner as it moves toward upper and a larger space may be ensured at the upper front of the slanted part 215 so that collision of the tray 300 with the upper case 213 becomes difficult. Here, the front face of the cover 214 is constructed such that the front face of the cover 214 covers this slanted part 215. Thus, when the cover 215 is closed, the front face is positioned near to the slanted part 215 while becoming parallel to the slanted part 215. As described above, corresponding to forming an obtuse angle between the upper face of the upper case 213 and the slanted part 215, the upper face of the cover 215 and the front face form an obtuse angle.

A bottom end of the slanted part 215 is placed at a far side than a guide member 230 and the outer front face of the lower case 211. That is to say, a distance between a back face of the medicine photographing device 200 and the lower end of the slanted part 215 is shorter than a distance between the back face of the medicine photographing device 200 and the outer front face of the guide member 230 and the outer front face of the lower case 211. By using this construction, it makes further easy for the user to insert the tray 300 into the upper inside space 240.

Furthermore, at an outer front face of the upper case 213, another slanted face 216 is disposed. More particularly, the slanted face 216 is disposed inside the near U-shape of the slanted part 215. The slanted face 216 is disposed in front of a first camera 410 (refer to Fig. 4) and protects the first camera 410. Thereby, when the user inserts the tray 300 into the upper inside space 240, displacements of a position and a direction of the camera 410 by a collision of the tray 300 with the first camera 410 may be prevented. As shown in Fig. 3, the slanted face 216 has a near rectangular shape and extends toward left and right directions of the medicine photographing device 200. Furthermore, as moving from top to bottom, a distance from the back face of the medicine photographing device 200 to the slanted face 216 becomes gradually decrease. That is to say, a horizontal position of the upper end of the slanted face 216 is nearer to the back face of the medicine photographing device 200 than a horizontal position of the lower end of the slanted face 216. Thereby, upon inserting, the tray 300 becomes difficult to colloid with the slanted face 216 and it becomes easy for the user to insert the tray 300 inner into the upper inside space 240.

As shown in Fig. 3, a pair of guide members 230 is disposed between the upper case 213 and the loading part 220. An outer lateral face of the guide member 230 extends toward the vertical direction and is positioned on the plane same with an outer lateral face of the upper case 213. Contradictory to the above, as shown in Figs. 3-5, an inner lateral face of the guide member 230 is inclined to the vertical direction, thereby, the slanted face 231 is formed. the slanted face 231 has a near rectangular shape and extends along to front-back directions of the medicine photographing device 200. Besides, the slanted face 231 faces to the upper inside space 240. Here, one of the slanted faces 231 is positioned at the right upper side of the loading part 220 while another one is positioned at the left upper side of the loading part 220. The slanted face 231 is formed such that the slanted face 231 becomes near to the center axis of the medicine photographing device 200 as going from top to bottom. That is to say, when viewed monoscopically, the horizontal position of the lower end of the slanted face 231 is nearer to the loading part 220 than the horizontal position of the upper end. Furthermore, the distance between two lower ends is set to be equal to or slightly larger than the width of the engagement part 223 of the tray support member 221. Particularly, the distance between lower ends of two slanted faces 231 is preferably to be 1-1.2 times of the width of the engagement part 223. Besides, when the medicine photographing device 200 is viewed monoscopically, a part of the lower end of the slanted face 231 is positioned at the location almost contacting to a periphery of a hole 222 of the tray support member 221. By the construction as described above, the user may become easy to place the tray 300 to the engagement part 223 of the tray support part 221.

### Section 2.2 Inner Construction of Medicine Photographing Device

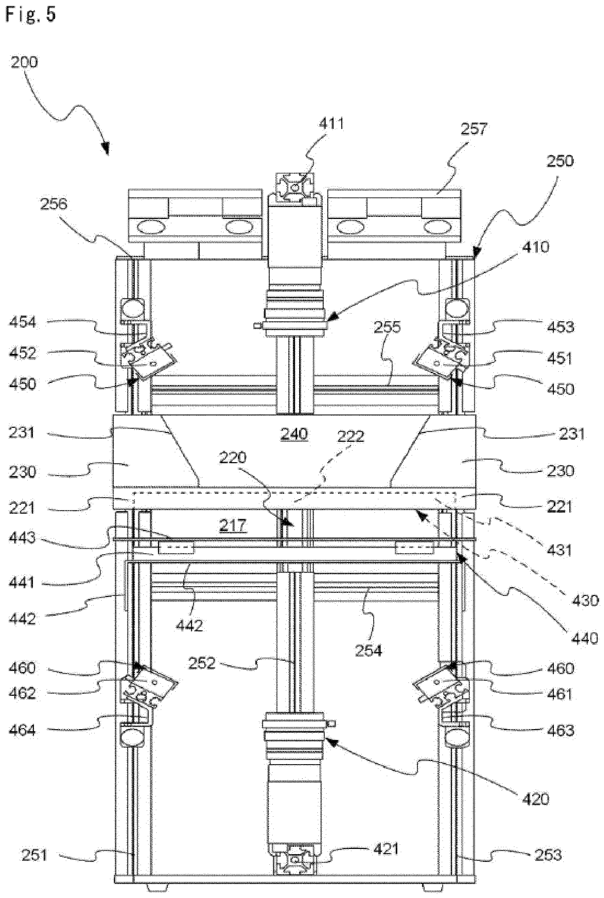

Fig. 4 and Fig. 5 illustrate the state that the case and the like of the medicine photographing device 200 are removed, i.e., Fig. 4 and Fig. 5 illustrate main members inside the medicine photographing device 200. Here, Fig. 4 corresponds a perspective view of the medicine photographing device 200 and Fig. 5 corresponds to a front view.

As shown in Fig. 4, at a back face side of the medicine photographing device 200, a flame 250 is disposed. Most of the main members of the medicine photographing device 200 are directly or indirectly fixed to the flame 250 for its construction. The flame 250 is constructed by a first column 251, a second column 252, a third column 253, a first beam 254, a second beam 255, a third beam 256 and a hinge 257. When viewed monoscopically, the first column 251 is disposed at a back face left side of the medicine photographing device 200; the second column 252 is disposed at the center of a back face; and the third column 253 is disposed at a back face right side. Besides, these first column 251, second column 252, and third column 253 are jointed by the first beam 254, the second beam 255 and the third beam 256. Furthermore, to the third beam 256 positioning on the top of the frame 250, a hinge 257 for rotating the cover 214 (refer to Fig. 3) is disposed.

As shown in Fig. 4 and Fig. 5, the medicine photographing device 200 comprises a first camera 410, a second camera 420, a first light source 430, a second light source 440, a third light source 450, a fourth light source 460, the tray support member 221 and the guide member 230. Among them, the tray support member and the guide member 230 are fixed to the frame 250 directly. The first camera 410, the second camera 420, the third camera 450 and the fourth light source 460 are fixed to the frame 250 via. support members. The first light source 430 is received in the tray support member 221 and is supported by the tray support member 221. While being not shown in the figures, the second light source 440 is fixed to the lower case 211 (refer to Fig. 2) via. a support member. As such manner, the main members in the medicine photographing device 200 are fixed to the frame 250 rather than the case 210. Thereby, the removal of the case 210 becomes easy and maintenance work becomes easy.

The tray support member 221 is disposed at the near center part of the medicine photographing device 200. Besides, at just near above the tray support member 221, the guide member 230 is disposed. Above the guide member 230, the third light source 450 is disposed. Furthermore, at higher position than the third light source 450, i.e. at near to the upper face of the medicine photographing device 200, the first camera 410 is disposed. At the position with a given distance lower from the tray support member 221, the second light source 440 is disposed. At the lower position than the second light source 440 and at near a lateral side of the medicine photographing device 200, the fourth light source 460 is disposed. At the position lower than the fourth light source 460, i.e. at near bottom face of the medicine photographing device 200, the second camera 420 is disposed. As such manner in the medicine photographing device 200, the first light source 430 and the third light source 450 compose an upper light source. Besides, in the medicine photographing device 200, the second light source 440 and the fourth light source 460 compose a lower light source.

As described above, the tray support member 221 is disposed at the near center part of the medicine photographing device 200 and above the space 217 for the notch part 212. Furthermore, at the bottom of the tray support member 221, the first light source 430 is fixed. The first light source 430 comprises a first ring lighting device 431. This first ring lighting device 431 is composed from a plurality of light emitting diodes (LED) disposed circumferentially. These LEDs are arranged so as to surround an outer periphery of the hole 222 of the tray support member 221 and are faced to the hole 222.

As shown in Fig. 4 and Fig. 5, at above the guide member 230, the third light source 450 is disposed. This third light source 450 is composed from a plurality of bar lighting devices, particularly from a first bar lighting device 451 and a second bar lighting device 452. The first bar lighting device 451 is fixed to the third column 253 via. the support member 453. Besides, the second bar lighting device 452 is fixed to the column 251 via. the support member 454. In other words, these first bar lighting device 451 and the second bar lighting device 452 are disposed at a lateral side of the medicine photographing device 200, more particularly at the lateral side beyond the loading part 220. As shown in Fig. 5, the first bar lighting device 451 and the second bar lighting device 452 are disposed in a direction parallel to a front-back direction of the medicine photographing device 200 and light emitting surfaces of the first bar lighting device 451 and the second lighting device 452 face to diagonal bottom, more particularly face to the loading part 220. Besides, distances from the first bar lighting device 451 and the second bar lighting device 452 to the position of the loading part 220 where the medicines are placed are larger than those from the first ring lighting device 431 to the position of the loading part 220 where the medicines are placed. Furthermore, the first bar lighting device 451 and the second bar lighting device 452 are placed at the positions nearer to the lateral face of the medicine photographing device 200 than the outer periphery of the hole 222 of the tray support member 221. With the arrangement of the third light source 450, large space may be ensured in the upper inside space 240 and it become easy for the user to place the tray 300
on the loading part 220.

The first bar lighting device 451 and the second bar lighting device 452 have polarization filters and are composed so that through the polarization filters diffused light can reach to the medicine. For further details, the first bar lighting device 451 and the second bar lighting device 452 are composed such that direct light from the first lighting device 451 and the second lighting device 452 may be cut by the polarization filter and the diffused light by the polarization filter reaches to the medicine after passing through the hole 222 of the tray support member 221, i.e. through the inside of a ring of the first ring lighting device 431. That is to say, the third light source 450 functions as a diffuse light source or an indirect light source. Thereby, an upper side of the medicine may be preferably lightened. The inventors have found that such construction of the third light source and the fourth light source makes it possible to take the photograph of prints provided on a surface of the medicine. Furthermore, the prints can be photographed clearly whether or not the medicine is a tablet or a capsule.

As shown in Fig. 4 and Fig. 5, at the lower direction from the second light source 440, the fourth light source 460, which is the kind same with the third light source 450, is disposed. This fourth light source 460 is composed from a plurality of bar lighting devices, particularly from the third bar lighting device 461 and the fourth bar lighting device 462. The third bar lighting device 461 is fixed to the third column 253 via. a support member 463. Besides, the fourth bar lighting device 462 is fixed to the first column 251 via. a support member 464. In other words, these third bar lighting device 461 and the fourth bar lighting device 462 are disposed at the lateral side of the medicine photographing device 200 as the first bar lighting device 451 and the second bar lighting device 452. Besides, the third bar lighting device 461 and the fourth bar lighting device 462 are disposed a in the direction parallel to the front-back direction of the medicine photographing device 200 as as the first bar lighting device 451 and the second bar lighting device 452. Light emitting surfaces of the third bar lighting device 461 and the second bar lighting device 462 face toward a diagonally upper direction, more particularly face toward the loading part 220. As similar to the relation between the third light source 450 and the first light source 430, distances from the third bar lighting device 461 and the fourth bar lighting device 462 to the position of the loading part 220 are larger than those from the second ring lighting device 441 to the position of the loading part 220. The third bar lighting device 461 and the fourth bar lighting device 462, as the relation between the first bar lighting device 451 and the second bar lighting device 452, also have polarization filters and are composed so that the diffused light can reach to the medicine through the polarization filters. For further details, the third bar lighting device 461 and the fourth bar lighting device 462 are composed such that the diffused light by the third bar lighting device 461 and the fourth bar lighting device 462 reaches to the medicines through the inside of a ring of the second ring lighting device 441. Thereby, a lower side of the medicine may be lightened so as to make the prints clear.

Now, according to the present embodiment, the third light source 450 and the fourth light source 460 are each composed from two bar lighting devices; however, in the other embodiment, four bar lighting devices may be disposed. In this instance, four bar lighting devices may be preferably arranged so as to form a rectangular shape so as to enclose the loading part 220.

At the upper position of the first lighting device 451 and the second bar lighting device 452, the first camera 410 is disposed. The first camera 410 is fixed to the second column 252 via. a fixture member 411. By this fixture member 411, the first camera 410 is located at the vertically upper position and is fixed so as to face the loading part 220. When the medicine photographing device 200 is viewed monoscopically from its top, a photographic area of the first camera 410 includes the hole 222 of the tray support member 221 and the inside of the ring of the first ring lighting device 431. Thereby, the first camera 410 may preferably take a photograph of the upper face of the medicine placed on the loading part 220, i.e., the image viewed just above the medicines.

At the lower position of the third bar lighting device 461 and the fourth bar lighting device 462, the second camera 420 is disposed. The second camera 420 is fixed to the second column 252 via. a fixture member 421. By this fixture member 421, the second camera 420 is located at the vertically lower position and is fixed so as to face the loading part 220. When the medicine photographing device 200 is viewed monoscopically from its bottom, a photographic area of the second camera 420 includes the hole 222 of the tray support member 221 and the inside of the ring of the second ring lighting device 441. Thereby, the second camera 420 may preferably take a photograph of the lower face of the medicine placed on the loading part 220, i.e., the image viewed just beneath the medicine. Both of the first camera 410 and the second camera 420 described above may take a color image.

### Section 3 Summary of Image Processing Program

The medicine photographing device 200 described above is, as depicted in Fig. 1, connected to the computer 500. The computer 500 comprises an image processing program 600 and by the image processing program 600 the computer 500 controls actions of the medicine photographing device 200. Particularly, the computer 500 controls lighting in the medicine photographing device 200 and makes the camera take the photograph of the medicine. Then, the computer 500 (receiver part 510) receives the photographed image from the medicine photographing device 200. The computer 500 (image processing part), for allowing workers to judge the medicine adequately, performs various processing and/or editing to the received image data and displays on the display device 530.

### Section 3.1 Basic Operation Procedure

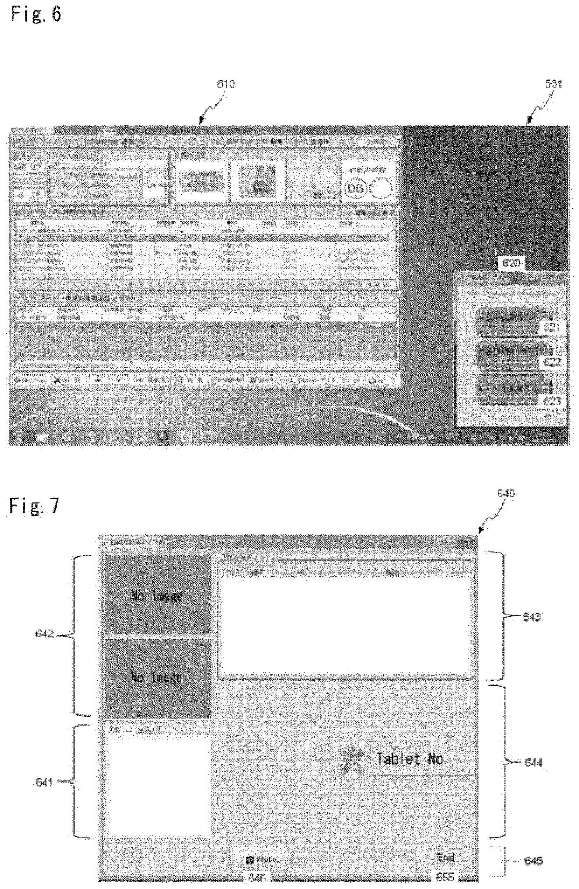

Fig. 6 illustrates a display screen 531 under the condition that the image processing program 600 is started. For further details, by the administration control supporting system 610 invokes the image processing program and the by image processing program a window 620 for a medicine judgement menu is displayed at a right lower position of the display screen 531. From the window 620, the judgement along to a purpose of the user will be initiated. As shown in Fig. 6, in the window 620, a "tablet image judgement" item 621, a "returned tablet image judgement" item 622, and a "loupe" item 623 are displayed. Here, in the window 620 the name "tablet judgement" is used; however, it is noted as a remark that the image processing program 600 may judge capsules as well as tables. The "tablet image judgement" item 621 may be used, as for one example, when specifying the medicines that the patients brought. The "loupe" item 623 may be used when providing an enlarged display of the medicines to which the user wants to see when the image processing program is running. Hereafter, the returned tablet image judgement will be explained.

### Section 3.2 Returned Tablet Image Judgement

The returned tablet image judgement is, as for one example, performed for specifying the returned medicine in order to reuse safely when the medicines once prescribed to hospitalized patients etc. in a medical institute have been returned without taken. For example, a plurality of kinds of the medicines were returned, pharmacists etc. first sort visually the medicines into the same kind. Next, the pharmacists etc., with respect to the medicines which have been decided to be the medicines in the same kind, as for the final confirmation, perform judgement and confirmation using a returned tablet image judgement function. Here, the returned tablet image judgement may be used not for specifying the returned medicines but also for the other use. As illustrated in Fig. 6, in the window 620 for the tablet judgement menu, the "returned tablet image judgement" item 622 is arranged. When the user selects the "returned tablet image judgement" item 622, as shown in Fig. 7, in the screen 531 the "returned tablet image judgement" window 640 is displayed. The returned tablet image judgement may be used under the assumption that all of a plurality of kinds of medicines expected to be judged is the same kind; however, when different kinds of medicines are mixed, various processing may be performed for making the user easy to aware contamination due to the different kinds of medicines as described below.

As shown in Fig. 7, the "returned tablet image judgement" window 640 comprises a first part 641, a second part 642, and a third part 643, a count and message display part 644 and an item display part 645.

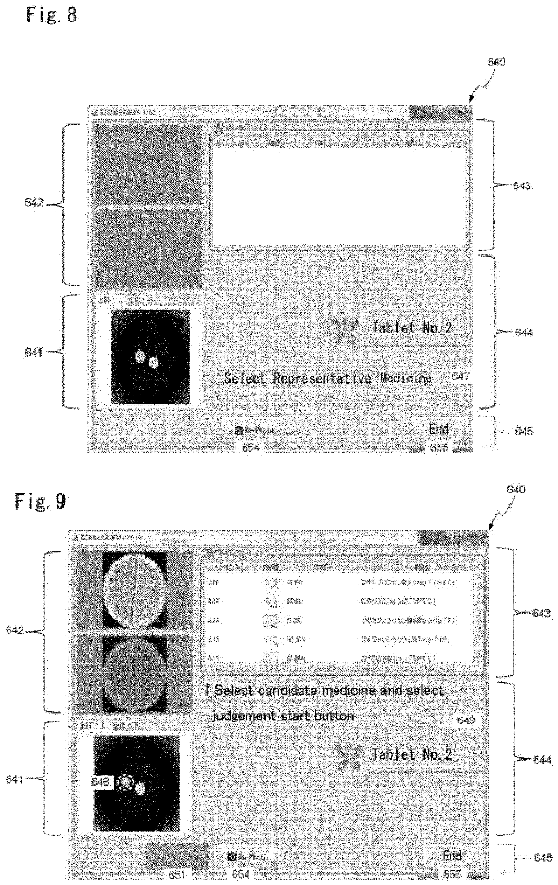

First, the user sets the tray 300 in which the medicines expected to be judged is contained to the medicine photographing device 200 and selects a "take photograph" item 646. Thereby, as shown in Fig. 8, the image of medicines photographed is displayed in the first part 641. That is to say, in the first part 641, the image photographing an area including a plurality of medicines is displayed. For this image, the image, which was photographed under the condition that the first light source 430 or the second light source 440 is turned on, may be used preferably. When the image is photographed under such lighting, contrasts between a background and the medicines tend to become larger. In this image, the area that includes a plurality of medicines photographed is present. At the upper area of the first part 641, a "view from top" tab and a "view from bottom" tab are arranged. When the user selects the "view from top" tab, the image of the medicines photographed from the top will be displayed in the first part 641. Alternatively, when the user selects the "view from bottom" tab, the image of the medicines photographed from the bottom will be displayed in the first part 641. In addition, the computer 500 performs automatically counts of the medicines and displays numbers of counted medicines in the count and message display part 644. Furthermore, the computer 500 displays a message 647 in the count and message display part 644. This message 647 demands the user to select one medicine (representative medicine) among the displayed medicines in the first part 641. When the user selects one medicine (representative medicine) displayed in the first part 641, the screen changes as shown in Fig. 9. Here, depending on settings, the computer 500 may, in place of the user, automatically select one medicine displayed in the first part 641 as the representative medicine.

Once the representative medicine is selected, an enlarged image of the selected representative medicine is displayed in the second part 642. At the upper area of the second part 642, the enlarged image of the selected medicine photographed from the top is displayed and at the lower area, the enlarged image of the selected medicine photographed from the bottom is displayed. Besides, when the representative medicine is selected, the computer 500 performs the judgement for the selected representative medicine selected by the user or the computer 500. Furthermore, in the first part 641, for easy understanding of the representative medicine selected by the user or the computer 500, the selected representative medicines are provided with a second label 648. After completion of automatic judgement by the computer 500, the computer 500 displays information of the medicines to be candidates as a list in the third part 643.

Each row in the list presents information of one candidate medicine. In a default setting, medicines having higher feasibilities for correctness in probability are presented in upper rows in the list. That is to say, the list display is sorted in a descendant order from top to bottom as the order from medicines with higher possibility to medicines with lower probability. Here, as for whether or not the medicines have high or low probability with respect to the correctness, a score obtained as the result of the search becomes an indicator. Particularly, when the score for coincidence probability about the medicine, that hits as results of the search, is high, the correctness probability becomes high; alternatively, if the score is low, the correctness score is low. In each row of the list, as the candidate medicine information, in turn from left, the score for correct possibility of the candidate medicine, the medicine image stored in the database, a stump or a printed code, and a medicine name. Here, by changing the settings, in place of the score, a rank for the correct possibility of this candidate medicine among all of the candidate medicines may be displayed. Furthermore, in addition to the medicine name, a code for specifying the medicine (for example, YI code) may be displayed. As described above, when the information of one medicine is displayed in one row altogether, the user may be recognized easily. Furthermore, in each of the rows, sizes of the medicines may be displayed.

Now, when a mouse cursor is moved near to the medicine image in the list displayed in the third part 643, the medicine image pointed by the mouse cursor may be provided in an enlarged display. As such, depending on the operation of the user, when one of the medicine images among a plurality of medicine images displayed on the third part 643 is provided in the enlarged display, the medicine image in that the user is now interest may be immediately seen in detail. In addition, and thereby, it becomes easy for the user to compare the medicine image displayed in the second part 642 with the medicine image displayed in the third part 643. Now, when the mouse cursor is moved apart from the enlarged image of the medicine, the enlargement of this image will be cleared so that the medicine image returns to an original size.

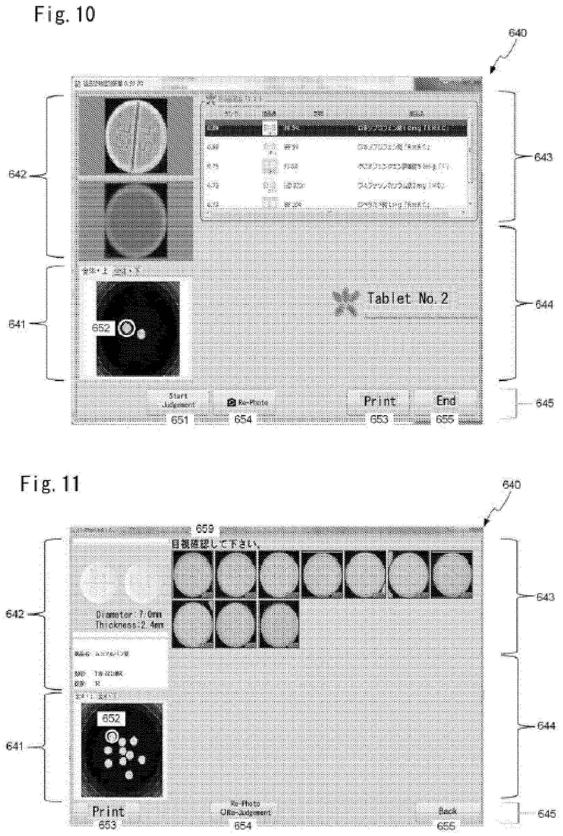

Thereafter, the user compares visually the medicine provided as an enlarged display on the second part 642 with the information of each candidate medicine listed in the third part 643 and the user selects a medicine that the user determined to be correct. When the user selects the medicine on the third part 643, as shown in Fig. 10, the row in which the selected medicine is indicated will be highlighted. In addition, in the first part 641, a third indicator 652, which shows completion of confirmation of the user, is provided to the corresponding medicine. In this state, the user may select the a "judgement initiation" item 651.

Fig. 11 illustrates the state that the user selects the "judgement initiation" item 651. For convenience of explanation, Fig, 11 illustrates the case that the returned tablet image judgement is performed to the returned medicines which are different from the returned medicine shown in Fig. 10. As shown in the figure, in the second part 642, a master image of the medicine selected in the third part 643 by the user, a medicine name, a stamp (or print), and a medicine number are displayed. In the third part 643, each of enlarged image of a plurality of medicines displayed on the first part 641 are displayed side by side. At a right bottom of the image of each medicine, a size of the medicine measured from the medicine image by the computer 500 is displayed.

As shown In Fig. 11, the computer further displays a message 659 on the third part 643. This message 659 demands the user to confirm visually the images of all of the medicines displayed on the third part 643. As the result of visual confirmation of the medicines displayed on the third part 643, when the user finds a different medicine, the user selects the medicine. When the different medicine is selected, in the first part 641, an indicator can be provided with the medicine corresponding to the different medicine. Thereby, the user can understand easily on which position the different medicines are placed and thus, the user can remove thereof without unmistakably. After the user removes the different medicines from the tray 300, the user selects a re-photograph item 654 and the above sequential processes may be repeated.

Here, the image processing program of the present embodiment has functions that the image processing program does not display each of a plurality of medicine images displayed on the first part 641 only on the third part 643 side by side but also sorts each of a plurality of medicine images That is to say, the image processing program of the present embodiment performs, when the representative medicine is selected among a plurality of medicines displayed on the display device 530 (first part 641), performs template matching (sometime called as pattern matching) between the representative medicine and each of a plurality of medicine images displayed on the display device (first part 641) and determines similarity degrees (matching rate). Here, the image of the representative medicine is the image that have been photographed by the medicine photographing device 200 and each image of a plurality of medicines are also the images photographed by the medicine photographing device 200. Therefore, the image processing program determines similarity degrees between images photographed by the medicine photographing device 200. The reason why the similarity degrees between images are determined as such is to sort the images of a plurality of medicines so as to find easily the contamination of the medicines etc. belonging to the different kinds when the pharmacists see visually the images of the medicines. Namely, the image processing program, based on the determined similarity degrees, displays the images of a plurality of medicines on the display device (the third part 643) after sorting.

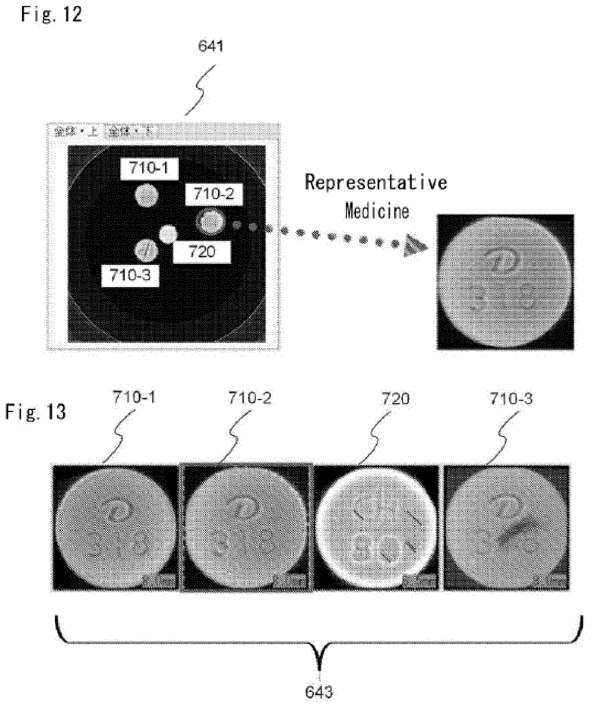

This aspect will be explained with referring to the drawings. In Fig. 12, four medicines are placed on the first part 641, and among them, three medicines 710-1, 710-2, and 710-3 are the same kind and the rest one medicine 720 is the different kind. Furthermore, the medicines 710-1, 710-2, and 720 are placed with facing the front while the medicine 710-3 is placed with facing the back. In this case, it is assumed that the medicine 710-2 is selected to be the representative medicine by the user.

In popular image processing program, nevertheless which medicine is selected as the representative medicine, as shown in Fig. 13, each of the medicines is displayed side by side on the third part 643 in descendant order from the medicine positioned upper in the first part 641. Particularly, the medicine 710-1 which is placed at the highest position in the first part 641 is displayed at the leftmost side in the third part 643. Furthermore, the medicine 710-2 which is placed at the second position in the first part 641 is displayed at the second from the left in the third part 643. The medicine 720 which is placed at the third position in the first part 641 is displayed at the third from the left. The medicine 710-3 which is positioned at the lower position is displayed at the rightmost side in the third position 643.

Contradictory to the above, the image processing program of the present embodiment as for one example, displays the images of a plurality of medicines by reordering (sorting) in the order of the low similarity degrees determined by the template matching on the display device (third part 643). That is to say, since the medicine 710-1 is the same kind with the representative medicine 710-2, the similarity degree to the medicine 710-2 becomes high to be about 0.9133. Since the medicine 710-2 itself is selected as the representative medicine, the similarity degree by the template matching becomes one. On the other hand, the medicine 710-3 is of the same kind with the the medicine 710-2 as the representative medicine; however, stains are adhered on a surface such that the similarity degree to the medicine 710-2 becomes somewhat low to be 0.8072. Since the medicine 720 is the different kind from the medicine 710-2 as the representative medicine such that the similarity degree to the medicine 710-2 becomes to be zero.

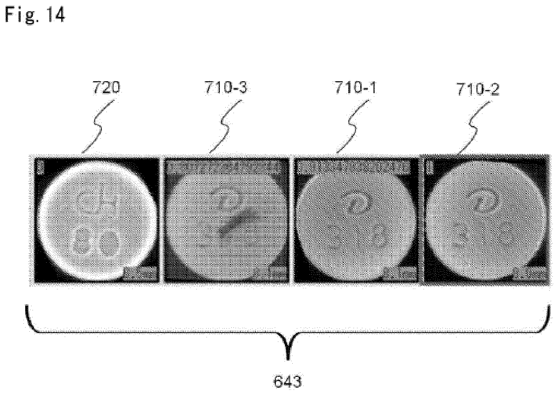

The image processing program of the present embodiment, as shown in Fig. 14, displays the medicine with the lowest similarity degrees 720 at the most left side on the third part 643. The image processing program also displays the medicine with the second lowest similarity degrees 710-3 at the second from the left side on the third part 643. The image processing program also displays the medicine with the third lowest similarity degrees 710-1 at the third from the left side on the third part 643. The image processing program also displays the medicine with the highest similarity degrees 710-2 at the rightmost side in the third part 643. Here, according to the present embodiment, an embodiment that a plurality of medicine images are sorted in the order in which the similarity degrees are low on the display device (third part 643) but not limited thereto. The image processing program may display on the display device (third part 643) a plurality of medicine images by sorting with the order of higher similarity degrees.

According to the present embodiment, since a plurality of medicines are displayed on the third part 643 by sorting based on the similarity degrees to the representative medicine, the user can find easily the medicine with the different kinds. That is to say, in the embodiment of Fig. 14, since the medicines are displayed with sorting from the lower order in the similarity degrees, the medicine of the different kinds will be displayed at the left side on the third part 643 with high possibility. Then, the user can find the medicines in the different kind easily by noticing particularly the medicines positioned at the left among a plurality of medicine images displayed on the third part 643. As the result, according to the present embodiment, the accuracy of the medicine judgement will be improved and the returned medicines can be reused safely. When a plurality of medicines is returned, there is the case in that first the pharmacists etc. classifies visually the returned medicines into the medicines of the same kind; however, there are fears that the contamination of the medicines of the different kinds may not be found only by viewing the medicines by eyes. Contradictory to this and according to the present embodiment, by displaying a plurality of medicines with sorting them based on the similarity degrees to the representative medicine, the pharmacist etc. can find easily the contamination of the medicine of the different kinds and can remove the medicine of the different kinds. Furthermore, even though the medicine is the same kind with the representative medicine, it is not adequate to reuse stained medicines. In this regard and according to the present invention, the similarity degrees of the stained medicine becomes relatively low such that there is the possibility that the display is provided at the left side on the third part 643. Therefore, the user can find the medicines with the stain easily by noticing the left side position. As the result and according to the present embodiment, the accuracy of the medicine judgement may be improved while returned medicines may be provided to reuse safely. Here, returning the the returned medicines to an original container or a tablet folding machine etc. for the reuse thereof requires quite high attention and high accuracy in the judgement. When the different medicines were returned to the container just in case, there would be the risk that quite severe event would be happened by mistakably administrated to the next patient and thus some medical institutes dispose the returned medicines for safe priority. However, as a countermeasure for recent increase of medical costs, such disposal becomes large social issue and the countermeasure becomes currently pressing need. In this regard, according to the present embodiment, since the medicine with the different kinds and the medicines with stains may be find easily, high accuracy medicine judgement may be realized. As the result, the returned medicine may be provided to reuse safely such that the present embodiment may become effective countermeasure for the recent increase of the medical cost.

Furthermore, the image processing program 600 of the present embodiment when displaying a plurality of medicine images on the third part 643, a function for aligning directions of a plurality of medicines is implemented. As for one example, the image processing program 600 displays the medicine images on the display device (third part 643) such that the directions of a plurality of medicine images are aligned as the representative medicine as a reference. For example, in the example shown in Fig. 14, the image processing program 600 rotates the direction of the medicine 710-2 selected as the representative medicine to the direction that the worker is easy to view (the direction that a letter D and numerals 318 at the front face of the medicine is easy to read). Then, the image processing program 600 rotates the direction of other medicines into the same direction with the direction of the medicine 710-2. By aligning the directions of a plurality of medicines, the worker can confirm whether or not the tablets with different kinds contaminate.

Furthermore, the image processing program 600 of the present embodiment when displaying a plurality of medicine images on the third part 643, a function for aligning front and back of a plurality of medicines is implemented. As for one example, the image processing program 600 displays the medicine images on the display device (third part 643) such that the front and back of a plurality of medicine images are aligned as a medicine front and back in the image of the representative medicine as a reference. For example, in the example shown in Fig. 14, the image processing program 600 displays the the medicine 710-2 on the display device (third part 643) so as to face the front such that the worker may see a letter D and numerals 318. Then, the image processing program 600 displays on the third part 643 other medicines with facing their fronts similarly to the medicine 710-2. For example, the medicine 710-3 were displayed on the first part 641 originally with facing its back and this processing makes the display with facing its front on the third part 643. By aligning front and back of a plurality of medicines as described above, the worker can confirm whether or not the tablets of different kinds contaminate.

Here, in the present embodiment, it may be possible to implement a learning function for the judgement processing to the return medicine. In conventional image judgement of the brought medicine, since various medicines may be brought, there were specifications that the tablet photographed was learned newly and was set as a master. On the other hand, the image judgement of the returned medicine is different from that of the brought medicines and the master is already present such that the specification for further learning and resetting as the master per se is not required. However, the returned medicine image judgement is different from the brought medicine image judgement and there is much practical use for photographing the same medicine at the same time. Therefore, when the representative medicine is selected among a plurality of medicines photographed, the medicine with less scratches and stains among the photographed medicines may be selected as the representative medicine. Depending on the particular case, there may be the case that the medicine with less scratches and stains than the current master is selected as the representative medicine and if such medicine is learned as the master, the processing accuracy of the medicine image judgement may be improved.

According to the present embodiment, the specification, which the similarity degrees obtained from the template matching are sorted in the descendant order or in the ascending order, may be additionally implemented. Besides, the scores by matching the currently stored as the master with other medicines and the scores by matching a medicine selected as the representative medicine with other medicines are compared. Then, averages of both scores are compared, and if the average of the representative medicine is higher, the determination that the representative medicine is superior than the master currently used, may be possible.

Next, if the representative medicine is excellent as the master, an announcement whether or not the representative medicine is replaced as the master (learning master information) may displayed on the display device 530. Particularly, on the "returned tablet image judgement" window 640, the message display for demanding the learned data update is provided, and the worker confirms the scores of the similarity degrees from the matching and then the worker may determine whether or not the master information is updated by the learning. Thereby, when the medicine which has less scratches and stains is selected as the representative medicine, the learning and update as the master may be continued so that the processing accuracy in the medicine image judgement may be improved sustainably.

### Section 3.3 Print of Results of Returned Tablet

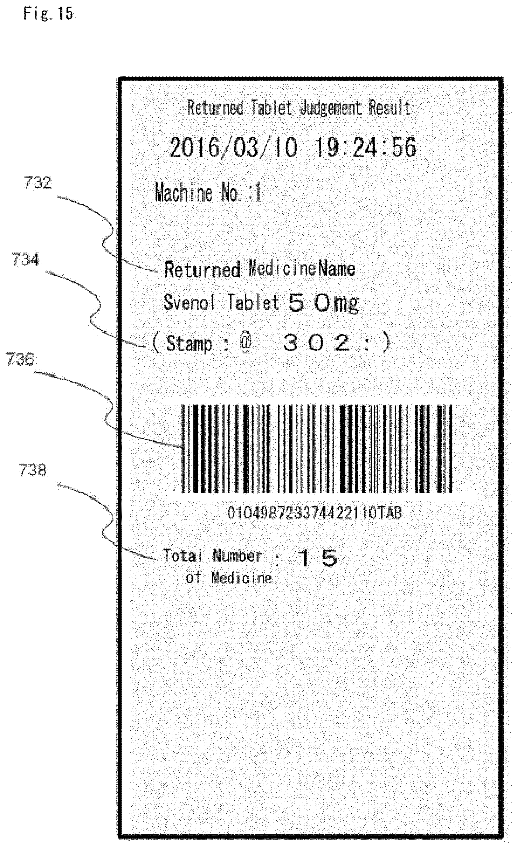

In Fig. 11, as the result of the visual confirmation by the worker, when it is confirmed that there is no contamination of the different medicines, the worker selects the "print" item 653. When the "print" item 653 is selected, the image processing program 600 prints the results of the judgement results for the returned medicines. Fig. 15 illustrates one example of the returned medicine judgement result printed.

The image processing program 600 prints, as shown in Fig. 15, the medicine name 732 returned, the stamp 734, a barcode 736 (for example, GS 1 code) and the total number of returned medicines 738, and the like as the results of the judgement of a plurality of medicines on a medium (for example, paper). Here, the image processing program 600 prints the judgement results including an identifier that indicates the medicines have been judged after a plurality of medicines were returned. Particularly, the image processing program 600 prints on the medium including the identifier that indicates the medicines has been judged after a plurality of medicines were returned at a region that shows a rot number of the medicine in the barcode 736.

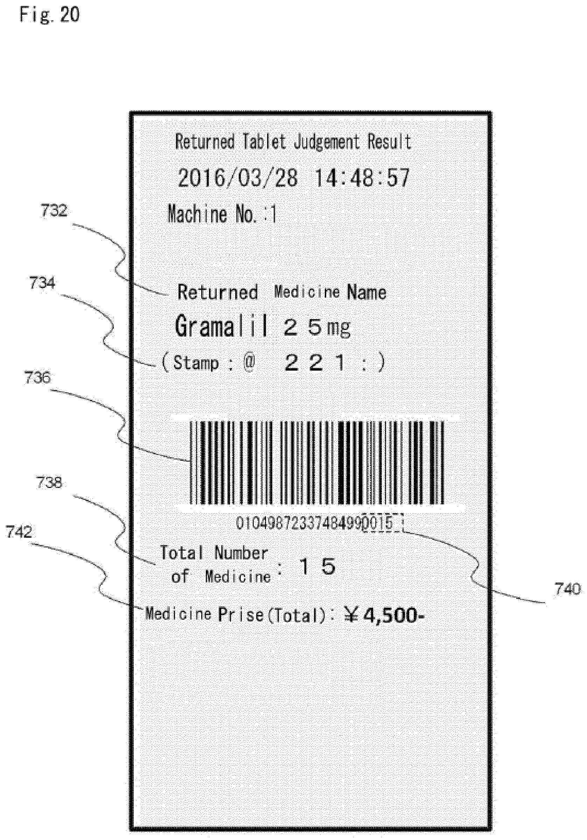

Furthermore, the image processing program 600 may print as shown in Fig. 20, the results of judgement of a plurality of medicines may be printed with incorporating the total number of the returned medicines 740 in the barcode 736 on the medium. Particularly, the image processing program 600 prints with incorporating the total number of the returned medicine 740 at the last 4 digit of the barcode 736 on the medium. According to the above, when the returned medicines are filled into the medicine folding machine, the total number of the returned medicines may be reflected to the medicine folding machine by reading the barcode 736 with a barcode reader of the medicine folding machine so that labor for inputting the total number of the returned medicines by manually may be omitted.

Furthermore, the image processing apparatus 600 as shown in Fig. 20, as the result of the judgement of a plurality of medicines, the total medicine price of a plurality of medicines judged may be printed. Particularly, the image processing program 600 calculates the total medicine price of a plurality of medicines judged by multiplying numbers of a plurality of medicines judged (total number 740) by each of the medicine prices of a plurality of medicines and then prints the total medicine price 742 on the medium. Each of the medicine prices of a plurality of medicines judged may be obtained by referring MD bank if "MD bank" (Trademark) from (Stock Company) YUYAMA MFG., CO., LTD. is installed to the computer 500 but not limited thereto.

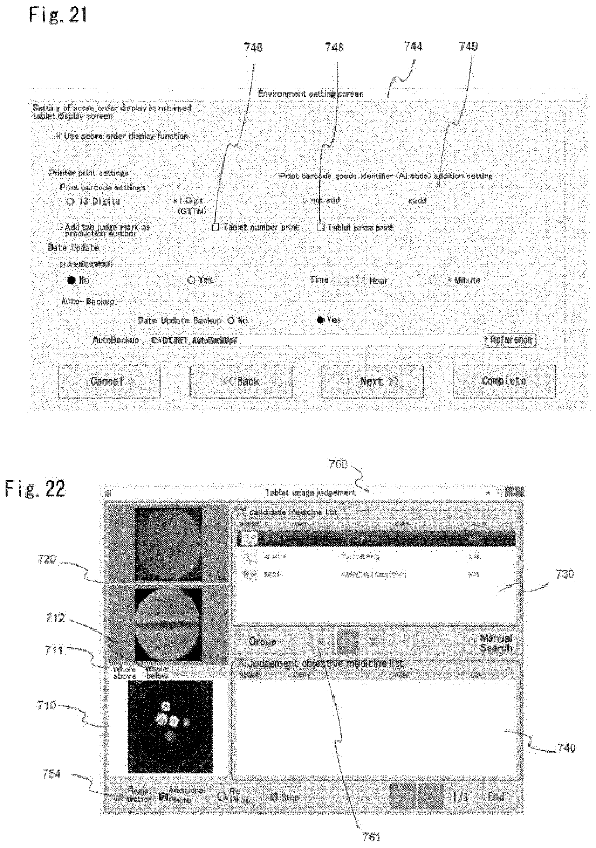

Here, the user may optionally set whether or not the total number of the judged medicines and the total medicine price are printed. Fig. 21 illustrates one example of an environment setting window for setting whether or not the total number of the judged medicines and the total medicine price are printed.

As shown in Fig. 21, in the environment setting window 744, a tablet number print check box 746 and a medicine price print check box 748 are displayed. If the user checks the tablet number print check box 746, the image processing program 600, when it prints the judgment results, prints the total number of the returned medicines 740 on the medium. In addition, if the user checks the medicine price print check box 748, the image processing program 600, when it prints the judgement results, prints the total medicine price 742 of the judged returned medicines 740 on the medium.

### Section 3.4 Flowchart of Image Processing Program

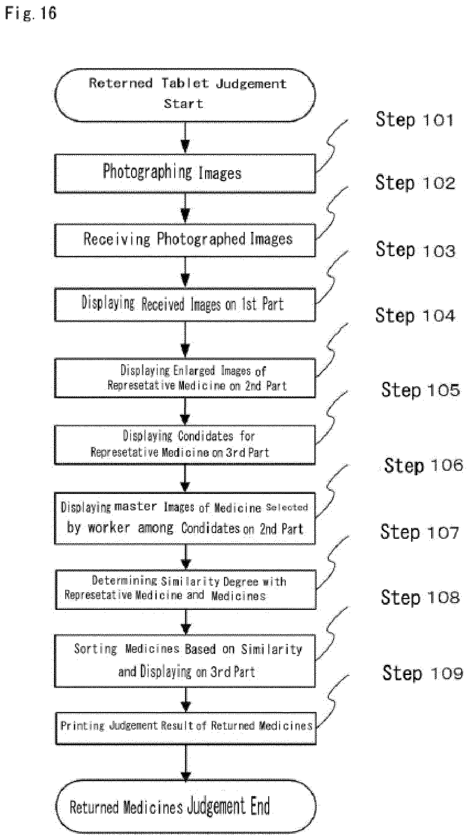

Next, the judgement processing of the returned medicines executed by the image processing program 600 will be described with referring to flowcharts. Fig. 16 is a flowchart of the processing executed by the image processing program. The image processing program 600, once a plurality of medicines is set to the medicine photographing device 200, first takes the photographs of the images photographing the region including a plurality of tablets by the medicine photographing device 200 (step 101). Then, the image processing program 600 receives with the receiver part 510 the images photographed by the medicine photographing device 200 (step 102). Then, the image processing program 600 displays the received images on the first part 641 (step 103). Further then, when the worker selects the representative medicine, the image processing program 600 provides the representative medicine image as an enlarged image on the second part 642(step 104). Here, the representative medicine may optionally be selected among a plurality of medicines by the image processing program 600.

Subsequently, the image processing program 600 displays the information of the medicine to become candidates for the judgement of the representative medicine on the third part 643 (step 105). Then, the image processing program 600, if the medicine determined correct by the worker is selected among the candidate medicines, retrieves the master image of the selected medicine from the database and displays thereof on the second part 642 (step 106).

Subsequently, the image processing program 600 obtains similarity degrees between the image of the representative image and each of a plurality of medicine images displayed on the first part 641 (step 107). Then the image processing program 600 displays the images of the plurality of medicines with sorting depending on the obtained similarity degrees on the third part 643 (step 108). Here, the image processing program 600, when displaying a plurality of images with sorting on the third part 643 in the step 108, may display the image of a plurality of medicines such that the directions of a plurality of medicine images are aligned with the image of the representative medicine as the reference. Besides, the image processing program 600, when displaying a plurality of images with sorting on the third part 643 in the step S 108, may display the image of a plurality of medicines such that front and back of the plurality of medicine images are aligned with the front and back of the representative medicine as the reference.

After the worker confirms that there is no contamination of the different kinds of medicines as the results of observations of a plurality of medicines displayed on the third part 643 side by side and when the "print" item 653 is selected, the image processing program 600 prints the judgement results of the returned medicines (step 109). The worker can fill the returned tablets into the medicine folding machine using the medium on which the judgement results are printed.

### Section 3.5 Judgement of Brought Medicines

Next, the judgement of the brought medicines that the patients brought will be explained. Fig. 22 illustrates one example of a picture image in the judgement of the brought medicines. By the user, the "tablet image judgment" item 621 is selected and the medicines that the patient brought have been judged, the "tablet image judgement" window 700 shown in Fig. 22 is displayed on the display device 530. The "tablet image judgement" window 700 roughly categorized into a first part 710, a second part 720, and a third part 740.

In the first part 710, the image that was photographed by the medicine photographing device 200 is displayed. For this image, the image photographed under the condition that the first light source 430 or the second light source 440 is turn on, may be used preferably. When the image is photographed under such lighting, contrasts between a background and the medicine tend to become larger. In this image, the area that includes a plurality of medicines photographed is present. At the upper area of the first part 710, a "view from top" tab 711 and a "view from bottom" tab 712 are arranged. When the user selects the "view from top" tab 711, the image of the medicines photographed from the top will be displayed in the first part 710. Alternatively, when the user selects the "view from bottom" tab 712, the image of the medicines photographed from the bottom will be displayed in the first part 710.

After the computer 500 takes the photographs, the computer 500 based on the images, automatically recognizes regions that each of the medicines occupies. When the user selects one of the medicines displayed on the first part 710, the enlarged image of the selected medicine is provided on the second part 720. At the upper area of the second part 720, the enlarged image of the selected medicine photographed from the top is displayed and at the lower area, the enlarged image of the selected medicine photographed from the bottom is displayed. Here, for leaving out the labor of user, and depending on the settings of the image processing program 600, the selection of the medicine may be performed automatically among the medicines after completion of the judgement.

When the judgement of the the selected medicine is completed, the computer 500 displays a list for candidate medicines obtained as the result of the judgement on the third part 730. Each row in the list displays information of one candidate medicine. In each row of the list as the candidate medicine information, the medicine image stored in the database, a stump or a printed code and a medicine name and score are displayed.

The user compares visually the medicine provided as the enlarged display on the second part 720 and information of each candidate medicine listed and displayed on the third part 730 and then the medicine that the user determined correct is selected. Particularly, the user selects the row in which the information of the correct medicine is displayed. Further then the user selects the "select" item 761 after the user reaches the belief that the medicine that the user itself selected is correct. Then, the information of the selected medicine is added to the fourth part 740. When confirmation work of one medicine is finished, the user selects the other medicine that the confirmation is not completed yet and the confirmation work will be performed to the selected medicine with the knack described above.

With respect to all of the medicines displayed on the first part 710, when the user completes all of the selection in the third part 730, or depending on the settings when all of the automatic judgement by the computer 500 is completed, if the user selects a "register" item 754, the judgement work of the medicine is completed. That is to say, when the user selects the "register" item 754, the judgement results are send to the administration control support system 610 and the "tablet image judgement" window 700 is closed.

### Section 3.6 Accumulation Work for Working Time and Costs Required for Judgement

Next, accumulation work for working time and costs required for judgement of the medicine will be explained. Fig. 23 is a flowchart of the accumulation work for working time and costs required for judgement of the medicine. Figs. 24, 25 are one example of screens for accumulation about the working time and costs required for the judgement of the medicine, respectively. As shown in Figs. 24, 25, in the window 750 for the tablet judgement menu, an "accumulation work" item 752 is included. When the user performs a click operation to the "accumulation work" item 752, the image processing program 600 displays the accumulation work window 754 on the display device 530.

In the accumulation work window 754, as a search condition for the accumulation work, a accumulation unit 755, an accumulation duration 756, an extract condition (apparatus number) 757, an output unit 758, an output object 759, and a "CSV output" item 760 are displayed. In the accumulation unit 755, the user can select at least one from "day designation", "month designation", "year designation". Furthermore, in the duration 756, the user can designate the duration for applying the accumulation work of the working time and costs required for the medicine judgement. In addition, in the extract condition (apparatus number), the user can designate the apparatus number as the object for performing the accumulation work.

Furthermore, the user in the output unit 758 can select the output of the accumulation result to be either an "output by user" or an "output by a day of week". In addition, the user can designate in the output object 759 the output object of the accumulation result to be either a "judgement" or a "return". Fig. 24 illustrates one example when the "judgement" is selected and the accumulation result for the judgement for specifying the medicines brought by the patient maybe outputted. On the other hand, Fig. 25 illustrates one example when the "returned" is designated and the accumulation result for the judgement for specifying the medicines returned may be outputted. Here, as shown in Fig. 25, in the output object 759, when the "returned" is designated, the user can input the "extract condition (medicine)"763 as the search condition. Particularly, the user can input in the extract condition (medicine) 763 a name of medicine and/or a stamp name as the search conditions.

In the condition that the search condition for the accumulation work is input, the user performs the click operation to the "CSV output" item 760, the image processing program 600, based on the search condition input through the accumulation work window 754, searches results of the medicine judgement corresponding to the search condition (step S202).

Subsequently, the image processing program 600, based on the results of the searched medicine judgements, accumulates the judgement time and costs (step S203). For example, the image processing program 600 measures the judgment time required for the medicine judgement and accumulates the measured judgement time or the judgement time for the medicine per tablet. Moreover, the image processing program 600 may measure the judgement time for judging a plurality of medicines required for judging a plurality of medicines and may accumulate the measured judgement time and the total medicine price of a plurality of medicines judged.

Subsequently, the image processing program 600 performs the CSV output based on the accumulation results (step S204). Particularly, the image processing program 600 accumulates when in the output object 759 the "judgement" is designated, the total time for judgement, an average working time of the judgement per medicine, and a medicine number judged and the like and creates the CSV (Comma Separated Values) file to store it in a storage device. As described above, by accounting the time and the like required to the judgement of the medicines brought by the patients and outputting as the CSV file, it becomes easier for the user to understand how much time is required for the medicine judgement.

On the other hand, the image processing program 600, when the "returned" is designated in the output object 759, accumulates the total return working time, the average return working time per one time, the return medicine number, and the total medicine price and generates the CSV (Comma Separated Values) file and then stores in the storage device, and the like. As described above, by accumulating the working time and the medicine prices relating to the returned medicine judgement and outputting as CSV, it becomes easier for the user to understand how much time is required for the returned medicine judgement and how much the medicine price is reduced by the medicine judgement.

That is to say, returning the returned medicines to the original container or the tablet folding machine for the reuse thereof requires considerably high attention and high accuracy judgement. When the different medicines were returned to the container just in case, there would be the risk that quite severe event would be happened by mistakably administrated to the next patient and some medical institutes dispose the returned medicines for safe priority. However, as a countermeasure for recent increase of medical costs, such disposal becomes large social issue and the countermeasure becomes currently pressing need. However, for performing the judgement, the worker costs for performing the judgement is required such that it may be preferred to consider balance between the reduced costs by the reuse of the returned medicines thought the judgement and the increasing costs by performing the judgement of the returned medicines. In this regard, according to the present invention, it becomes easier for user to understand that how much time (cost) is required for the judgement of the returned medicines and mow much the medicine price is reduces by the returned medicine judgement. As the result, the judgement working of the returned medicines may be adequately practiced.

Here, the above embodiment, the example in which the accumulation results of the working time relating to the medicine judgement are output in CSV, but not limited thereto, the image processing program 600 may display the accumulation results on the display device 530. Fig. 26 is a drawing showing one example of an accumulation result screen such as judgement time required for judging the medicines. The example of Fig. 26 corresponds to an example of the accumulation result screen of the judgement time required to specify the medicine that the patient brought or the returned medicines, and the like in the case that the above "tablet image judgement" item 621 or the "returned tablet image judgement" item 622 is selected. Besides, in the example of Fig. 26, the user may select as the search condition a user information search item 765, a patient information search item 767 and a medicine information search item 769. The user may input a user ID or user name as the search key to the user information search item 756. Besides, the user may input a patient ID or patient name as the search key to the user information search item 758. Furthermore, the user may input a medicine name or medicine stamp name as the search key to the user information search item 760. The image processing program 600 may display the results accumulated based on the user information search item 765, the patient information search item 767, or the medicine information search item 769 on the display device 530.

Particularly, as shown in Fig. 26, in the accumulation result screen 762, an average judgement time 764 per medicine, an average judgement time per day 766, an average medicine number per day 768, and a list 770 of the judgement corresponding to the search condition. In the list of the judgement 770, a judgement No, a machine number of the medicine photographing device 200 for performing the judgement, a date for performing the judgement, a user name for performing the judgement, a patient name which brought the medicine, the medicine number judged, the time required for the judgement and the WEB judgement cooperation No etc. Here, the time required for the judgement means the time from photographing newly the medicines by the medicine photographing device 200 to the registration of the judgement (for example, until the "registration" item 754 is selected) and is measured by the image processing program 600. As described above, by displaying the time required for the medicine judgement and the like after the accumulation, it becomes easier for the user to understand how much time is required for the medicine judgement.

Now, Fig. 27 is a drawing showing one example of the accumulation result screen for the judgement time, and the like for judging the medicine. Fig. 27 is one example of the accumulation result screen of the judgement time, and the like required for judging a plurality of returned medicines in the case that the "returned tablet image judgement" item 622 is selected.

Particularly, as shown in Fig. 27, in the accumulation result screen 762, an average working time per one return work 772, an average working time per day 774, an average returned medicine number per day 776, a medicine price (total accumulation) 778, and a list 780 of the judgement corresponding to the search condition are displayed. In the list 780 of the judgement, a judgement No, a machine number of the medicine photographing device 200 for performing the judgement, a date for performing the judgement, a user name for performing the judgement, a medicine name returned by the judgement, a medicine number returned by the judgment, a time required for the judgement and a medicine price (total) of the medicine returned by the judgement, and the like. Here, the time required for the judgement means the time from photographing newly the medicines by the medicine photographing device 200 to the registration of the judgement (for example, until the "print" item 653 is selected) and is measured by the image processing program 600. As described above, by displaying the time required for the medicine judgement, the medicine price of the medicines returned by the judgement, and the like after the accumulation, it becomes easier for the user to understand how much time is required for the medicine judgement and how much the medicine price is reduced.

### Section 3.7 Massage in Medicine Judgement

Hereinafter, a display of a message upon performing the medicine judgement will be explained. Here, an example, in which the "tablet image judgement" item 621 is selected and the medicines that the patient brought are judged and the message is displayed, will be explained. However, not limited thereto, the similar message may be displayed when the "returned tablet image judgement" item 622 is selected and the returned medicines are judged.

Fig. 28 is a flowchart of the message display work. Figs. 29-31 are drawings of one example of a condition that displays the message relating to the medicine judgement, respectively. As described above, the "tablet image judgement" item 621 is selected by the user and then the medicines that the patient brought has been judged, the image processing program 600 displays the "tablet image judgement" window 700 shown in Fig. 29 on the display device 530 (step S301).

In the first part 710, the image which is photographed by the medicine photographing device 200 is displayed. When the user selects one of the medicines displayed on the first part 710, the image of the selected medicine is provided on the second part 720 as the enlarged display. At the top of the second part 720, the image of the selected medicine photographed from above is provided as the enlarged display and at the bottom the image of the selected medicine photographed from below is provided as the enlarged display. Now, for omitting labor of the user, depending on the settings of the image processing program 600, the selection of the medicines may be performed automatically by the computer 500 among the medicines that the judgement is completed.

When the judgement of the selected medicine has been completed, the computer 500 displays on the third part 730 the list of the candidate medicines obtained as the result of the judgement. Each of the row in the list displays information of one candidate medicine. In each row in the list, as the candidate medicine information such as the medicine image, the stamp, the medicine name and the score, and the like that are stored in the database are displayed.

Here, the image processing program 600 displays as shown in Fig. 29 an identifier 782 which remarks presence of the message when displaying the information of the medicines to be the candidates for the medicine judgement if the medicines to be the candidate are associated with the message (step S302), In this instance, three medicines to be the candidate are displayed on the third part 730 and the messages are associated with two medicines among them. On the other hand, with respect to the medicines with no identifier 782, it is shown that no message is associated.

The image processing program 600 displays the message item 784 as shown in Figs. 30, 31 when the medicine to be the candidate or the identifies 782 is selected or when the mouse cursor comes near with respect to the medicine to be the candidate or the identifier 782 (step S303). According to the present embodiment, when the mouse cursor moves near to the image of the medicine in the list displayed on the third part 730, the image of the medicine to which the mouse cursor points is provided as the enlarged display and the message item 784 is displayed. In the examples of Figs. 30 31, the message item 784 notices that there are a plurality specification differences for the selected medicine. The message item 784 may display various items depending on the medicines such as presence of a plurality of differences in the stamp of a back face as well as the presence of differences in the specification. In addition, in the case that the "returned tablet image judgement" item 622 is selected, for example, various message item such as "Lasix changes color and dispose without return", and the like.

As described above, when the image displayed on the third part 730 is presented as the enlarged image depending on operation of the user, details of the medicine image that the user is now interested in may be immediately viewed. In addition, and thereby, it may become easier for the user to compare the medicine image displayed on the second part 720 and the medicine image displayed on the third part. Now, when the mouse cursor moves apart from the enlarged medicine image, the enlargement of the image is released and the medicine image returns to the original size.

The user compares visually the medicine provided in the enlarged display on the second part 720 and each of the candidate medicines displayed as the list on the third part 730 and selects the medicine that the user determines correct. At this instance, according to the present invention the identifier 782 that notices the presence of the message with the medicine to be candidate is displayed on the display device 530 and further the message item 784 is displayed on the display device 530 such that the user may select the correct medicine after considering contents of the message item 784.

Here, the user may browse, register, edit, and delete the message item 784. Fig. 32 is a drawing of one example of the screen for editing the message item with respect to the medicine judgement. As shown in Fig. 32, when the user brings the mouse cursor near to the medicine to be the candidate or the identifier 782 and then performs a right click operation, a menu screen 786 is displayed on the display device 530. In the present example, since it is premised that the message item 784 is already set to the medicine to be the candidate, an "edit" menu is displayed on the menu screen 786. On the other hand, when the message item 784 is not set yet to the medicine to be the candidate, a "register" menu is displayed on the menu screen 786.

When the "edit" menu is selected in Fig. 32, a message item entry screen 788 is displayed on the display device 530. The user may enter the message item into an entry field 790 of the message item entry screen 788 by manual input. Besides, when the entry field in the state that anything is not entered is pointed by the mouse cursor and for example an Enter key of a keyboard is clicked, the message item selection screen 792 is displayed on the display device 530. In the message item selection screen 792, a message item list 794 recorded beforehand is displayed. Fig. 32 illustrate two message items are recorded beforehand; however, the number of registrations of the message items beforehand may be optionally. The user may select an adequate one and then performs a click operation to a selection item 796 and may reflect the selected message item to the entry field 790. Alternatively, the user may perform a click operation to a register item 798 of the message item entry screen 788 and may set the message item entered in the entry field 790 as the message item of the medicine to be the candidate. Furthermore, the user performs a click operation to a delete item 799 of the message item entry screen 788 and may delete the message item 784 set to the the dug to be the candidate.

### Section 3.8 Association of Patient Information in Brought Medicine Judgement

Next, in the case that the medicine that the patient brought is judged, an embodiment for correlating information relating to the patient that brought the medicine to the judgement result will be explained. Fig. 33 is a flowchart for a patient information entry work. Fig. 34 illustrates one embodiment at the condition that the information relating to the patient of the brought medicine is displayed in the screen for performing the judgement of the medicine.

The image processing program 600, in the case that the medicine brought by the patient is judged, makes the computer 500 execute the step for displaying a judgement screen for judging the brought medicine of the patient on the display device 530 (step S401). As the result, as shown in Fig. 34, a "tablet image judgement" window 700 is displayed on the display device 530. According to the present embodiment, in the "tablet image judgement" window 700, a first part 710, a second part 720, a third part 730 and a fourth part 740 as well as a fifth part 1100 for displaying the patient information are displayed. In the fifth part 1100, a patient name, sexuality, a hospital ward and a diagnosis and treatment department and the like are displayed. In addition, a patient attribute item 1102 are disposed in the fifth part 1100.

The image processing program 600 makes the computer 500 execute the step for receiving information about the patient that brought the medicine through a judgement screen displayed on the display device 530 (step S402). Particularly, the image processing program 600, when a patient attribute item 1102 is selected with a click operation by the user, displays a screen for inputting the patient attribute (attribute entry screen 1110). Fig. 35 is a drawing that shows one example of a screen for inputting the attribute of the patient that brought the medicine. To the attribute input screen 1110, the information such as a patient number, a patient name, a date of the judgement, a hospital ward, a diagnosis and treatment department, and the like may be input. The attribute input screen 1110 is implemented so as to allow the user to edit the patient information reflected from the database. For example, the user points the mouse cursor to a field for entering the hospital ward and then performs a click operation to the Enter key of the keyboard, a hospital ward selection screen 1112 is displayed on the display device 530. In the hospital ward selection screen 1112, a hospital ward list 1114 registered beforehand is displayed. When the user selects an adequate one from the hospital ward list 1114 and then performs a click operation to the selection item 1116, the selected hospital ward may be reflected to the attribute input screen 1110. Items other than the hospital ward may be similarly selected from the list. In addition, the attribute input screen 1110 is also implemented so as to allowing the user to input manually the patient information in the attribute input screen 1110. The image processing program 600, when a registration item 118 of the attribute input screen 1110 is selected by a click operation, displays the patient information input in the attribute input screen 1110 on the fifth part 1100 (step S403)

On the other hand, when the patient information is not stored in the database, the patient information may be registered newly. Fig. 36 is a drawing of one example of the screen for inputting the attribute of the patient that brought the medicine. When the patient information is not recorded in the data base, the patient information is registered through the patient information register screen 1120. In the patient information register screen 1120, fields for registering the patient number, the patient name, birthday, sexuality, and the like are provided. The user may input the patient information to each item and then may perform a click operation to the registration item 1122 to register the patient information to the database.

In addition, in the present embodiment, the image processing program 600 performs the computer 500 execute the step for correlating the judgement result of the brought medicine with the information relating to the patient brought the medicine (step S404). Particularly, the image processing program 600 may store the judgement result of the medicine that the patient brought and the patient information with correlating each other in the storage device, and the like. Thereby, using the patient information such as for example the patient name, and the like as the search key, the judgement result of the medicine brought by the patient may be searched such that usability of the user may be improved.

### Section 3.9 Usage Input in Brought Medicine Judgement

Next, usage input in the case that the medicine brought by the patient is judged will be explained. Fig. 37 is a flowchart of usage input work. Fig. 38 is a drawing of one embodiment for the judgement screen in the judgement of the medicine brought by the patient. The image processing program 600, in the case for the judgement of the medicine brought by the patient, makes the computer 500 execute the step for displaying the judgement screen for judging the brought medicines by the patient on the display device 530 (step S501). As the result, as shown in Fig. 38, on the display device 530 the "tablet image judgement" window 700 is displayed. In the "tablet image judgement" window 700, an RP switching item 1230 is provided.

In addition, in the "tablet image judgement" window 700, a first part 710, a second part 720, a third part 730, a fourth part 740 and further a sixth part 1200 for displaying usage information are provided. In the sixth part 1200, information of the medicine to be an object of the judgement may be displayed. Besides, in the sixth part 1200, a usage selection item 1202 is provided.

The image processing program 600, through the "tablet image judgement" window 700 displayed on the display device 530, makes the computer 500 execute the step for receiving input of the usage for the medicine that the patient brought (step S502). Particularly, when the usage selection item 1202 is selected by a click operation, the image processing program 600 actuates (displays) a screen for selecting the usage. Fig. 39 is a drawing of one example of the screen for inputting the usage of the medicine that the patient brought via. the judgement screen for judging the medicine that the patient brought. In the usage selection screen 1210, when a "after each meal" item 1212 is selected by the user, in the sixth part 1200, check marks are added to items of "morning", "lunch" and "evening" and then an item "after meal" becomes active. Besides, when the user selects a "after morning and evening meal" item 1214, in the sixth part 1200, check marks are added to a "morning" and a "evening" items and then a "after meal" item becomes active.

Furthermore, the user may also select the usage not being present in the usage selection screen 1210. Fig. 40 is a drawing of one example for a screen for inputting the usage of the medicine that the patient brought via. the judgement screen for judging the medicine that the patient brought. As shown in Fig. 40, when the item "after meal" displayed on the sixth part 1200 is selected by the click operation, the usage list 1220 is displayed. In the usage list 1220, kinds of usage such as "before meal", "between meal", "just before meal" are displayed. The user may select optional usage from the usage list 1220 and may reflect the selected usage to the sixth part 1200. According to the present embodiment, since the usage may be input through the "tablet image judgement" window 700, the labor of the user for input settings of the usage may be omitted.

In addition and according to the present embodiment, the image processing program 600 makes the computer 500 execute the step for correlating the information about the usage of the medicine that the patient brought (step S504). Particularly, the image processing program 600 may store the judgement result of the medicine brought by the patient with correlating the usage of the medicine in the storage device, and the like or may print the judgement result and a method of medicine on the medium.

Here, there are many cases that the brought medicine were prescribed in a plurality of pharmacies such that the judgement of the medicines that have been prescribed in each hospital may be performed daily. According to the present embodiment, the setting is made such that the switching between active and inactive of the RP switching may be done every time the user performs the click operation to the RP item change item 1230. For the background, even though the patient is one; since it may be seen a doctor at a plurality of hospitals and in such cases, the patient brings the medicines that have prescribed in each of every hospital. In such case, by providing RP switching item 1230, the user may designate the switching of the RP so that judgement of the medicines that have been prescribed at each of every hospital may be performed and may be managed.

For example, if it is needed that the medicine prescribed in the hospital A is judged as Rp1, and next the medicine prescribed in the hospital B is judged as Rp2, RP switching may be activated by performing the click operation to the RP switching button RP. Thereby, the judgement of the brought medicines may be performed for Rp set to every hospital and the medicines of each of every hospital may be managed.

Fig. 41 is a flowchart of RP switching. In the system for judgement report to which the judgement data is sent by this apparatus, it is designed that styles of the report may be freely edited. For example, using the case as a example that if the medicines brought by one certain patient has been prescribed from the hospital A and there are residual quantities of morning 20 tablets, noon 10 tablet and evening 10 tablets. Furthermore, there are residual quantities of ones prescribed in the hospital B to be morning 5 tablets and evening 5 tablets, methods of operation and data input will be explained bellow.

When RP switching button 1230 is selected by the click operation of the user (step S505), the image processing program 600 starts RP switching. First, the tablet image judgement screen set by Rp and the judgement work is started (step 506-508). When there are residual quantities of morning 20 tablets, noon 10 tablets and evening 10 tablets as the medicines from the hospital A, first the work is started from judging the medicines 20 tablets for Rp1 morning in a dish. Then, the usage selection button "morning" is pressed and the judgement Rp1 morning is completed. Furthermore, the medicines used in the judgement are removed and subsequently, the medicines of 10 tablets for noon are placed thereon and then an additional photographing button is pressed and further then the usage selection button "noon" is pressed. Further then, the medicines of 10 tablets for evening are placed thereon and the additional photographing button "evening" is pressed. These operations are same with ones that has already explained in Figs. 38-40. As described above, first the judgement of the medicines from the hospital A is completed. (step 902).

Next, by pressing Rp switching button, the process transfers to the judgement of the prescription from the hospital B (step 903). As the medicines from the hospital B, when there are the residual quantities of morning 5 tablets, evening 5 tablets, the morning medicine 5 tablets are placed thereon and the additional photographing button is pressed. After taking the photograph, the usage selection button "morning" is pressed. Next, the medicines for evening 5 tablets are placed thereon and the additional photographing button is pressed, and then the usage selection button "evening" is pressed. As described above, the medicines from the hospital B is completed (step 508). After the entire judgements have been completed and by pressing the registration item 122 is selected with the click operation, the patient information by each of every hospital may be newly recorded (step 509).

Here, in the system for making the judgement report of the present embodiment, every operation done by the above each operation, that is to say, the data may be sent by Rp, by dish, and by usage so that it may be possible to design freely the judgement report. When there are further a plurality of hospitals, similarly, the operation from the steps 506 to 508 will be performed about the hospital C, D, E,---. In addition, when the capacity of the dish allows to place thereon only 15 tablets at most and when judging the medicine for morning of 20 tablets as described in Fig. 41, first the 15 tablets are placed on the dish and a new photographing button is pressed to take the photograph. Then, the 15 tablets are removed and residual 5 tables are placed on the dish and thereafter next the additional photographing button is pressed. Further then the usage selection button "morning" is pressed to complete and to make recognize on the system the judgement of Rp1 morning. Then, the medicines used for the judgement are removed, and subsequently, the noon 10 tablets are placed thereon and then the additional photographing button is pressed followed by pressing the usage button "noon". Further then the evening 10 tablets are placed thereon and then the additional photographing button is pressed followed by pressing the usage selection button "evening". As described above, the present judgement system 100 may be set such that the judgement work may be continued if the maximum content of the dish is exceeded.

### Section 3.10 Maintenance Function

Next, a function for changing the setting items of the medicine judgement from a menu screen of the medicine judgement will be explained. Fig. 42 is a flowchart of maintenance work. First, when the medicines are judged, the image processing program 600 makes the computer 500 execute the step for displaying the menu screen about the medicine judgement on the display device 530 (step S601). Thereby, a window 750 of the tablet judgement menu in Figs. 24, 25 is displayed on the display device 530. Subsequently, the image processing program 600 through the window of the tablet judgement menu makes the computer 500 execute the step for receiving change input for the setting items about the medicine judgement (step S602). Particularly, when a "maintenance" item 753 of the tablet judgement menu in Figs. 24, 25 is selected by the click operation, the image processing program 600 displays a setting change screen 1300 shown in Fig. 43 on the display device 530. Fig. 43 is a drawing of one example for changing the settings about the medicine judgement.

As shown in Fig. 43, on the setting change screen 1300, a function setting item 1310 for setting use/non-use of various functions about the medicine judgement is displayed. Besides, as shown in Fig. 43, setting items about the medicine judgement includes the usage of the medicines which are objective of the judgement such that in the setting change screen 1300 a usage setting item 1320 for setting the usage of medicines is displayed. The user may set the use/non-use of the various functions by selecting through the click operation items for setting various functions in the function setting item 1310. Furthermore, when a usage registration screen activation item 1322 is selected through the click operation, the usage registration screen 1330 is activated (displayed). Fig. 44 is a drawing of one example of the screen for registering the usage of the medicines.

As shown in Fig. 44, in the usage registration screen 1330, a usage registration item 1332, a registration item 1334, a list 1336, a confirmation item 1338 and a deletion item 1340 are displayed. The user, for example, when it wants to register usage such as after every meal, enters check marks to the items of "morning", "noon", and "evening" in the usage registration item 1332 under the condition that the item of "after meal" is in active, and selects the registration item 1334 with the click operation. Then, as shown in Fig. 44, the usage "after every meal" is displayed in the list 1336. The image processing program 600, when the confirmation item 1338 is selected with the click operation under this condition, registers the changed setting item, i.e., the usage "after every meal" (step S603).

Here, in this example, it may be possible to register the usage of six, but not limited thereto, the number of registrations may be optional. For example, the user enters the checks to the items "morning" and "evening" and changes the item "after meal" to the item "before meal" and then performs the click operation to the registration item 1334 so that the usage "before morning and evening meal" maybe registered. When the user also performs the deletion item 340 upon pointing the mouse cursor to the usage already registered, the corresponding usage is deleted and the corresponding part of the list 1336 becomes blank. According to the present embodiment, the user may easily change various settings such as the usage of the medicines through the window 750 of the tablet judgement menu.

### Section 3.11 Manual Search of Judged Medicine

Next, a manual search of the judgement medicines will be explained. Fig. 45 is a flowchart of the manual search work of the judgement medicines. when the medicines are judged, the image processing program 600 makes the computer execute the step for displaying a judgement screen for judging the medicines on the display device 530 (step S701). Thereby, a "tablet image judgement" window 700 as shown in Fig. 46 is displayed on the display device 530. Fig. 46 is a drawing of one example of a screen for the manual search via. the judgement screen for judging the medicines. In the "tablet image judgement" window 700, a first part 700, a second part 720, a third part 730, a fourth part 740 and furthermore a "manual search" item 1400 for activating the screen for searching the medicines manually is provided.

The image processing program 600 through the "tablet image judgement" window 700 makes the computer 500 execute the step for displaying the screen for searching the medicines manually on the display device 530 (step S702). Particularly, when the worker performs the click operation to a "manual search" item 1400, the image processing program 600 displays on the display device 530 a medicine search screen 1410 shown in Fig. 47. Fig. 47 is a drawing of one example of the screen for manual search of the medicine.

As shown in Fig. 47, in the medicine search screen 1410, a search condition input item 1420, a medicine display item 1430, a search result display item 1460 are displayed. In the search condition input item 1420, as the condition for searching the medicines, the "stamp" or the "medicine name" may be allowed to be selected. The worker selects either the "stamp" or the "medicine name" and then input a search condition in the search box 1422 and further then performs the click operation to the search item 1424. Hereupon, in the search result display item 1440, a list of the medicines matched to the search condition is displayed. When the worker selects any one medicine from the list of the search result display item 1440, the image of the selected medicine is displayed on the medicine display item 1430.

Here, within the medicines displayed in the list of the search result display item 1440, the medicines to which the message item is set are displayed together with the identifier 1441 that acknowledges the presence of the message item. For example, when the medicine that the message item is registered to a particular medicine through the message item entry screen 788 of Fig. 32 is selected in the search result display item 1440, the registered message item is displayed on the message item display item 1460. By displaying the message item on the medicine search screen 1410 as the present embodiment, a judgement mistake by the manual search of the worker will be avoided.

Hereupon, in the message item display item 1460, the message item edit item 1462 is disposed. The worker may edit by performing the click operation to the message item edit item 1462 the message item already set to the selected medicine. In addition, the worker may by performing the click operation to the message item edit item 1462 set a new message item when the message item is not set yet to the selected medicine.

In the condition that the medicine is selected in the search result display item 1440, when the worker performs the click operation to the addition item 1450, the tablet number input screen 1500 is displayed on the display device 530. Fig, 49 is a drawing of one example of the screen for manual search of the medicine. As shown in Fig. 48, in the tablet number input screen 1500, a medicine display item 1510 for displaying the image of the selected medicine, a tablet number input item 1520 for inputting the tablet number of the medicine and an "OK" item 1530 for adding the medicine to the fourth part 740 are displayed.

When the worker inputs the tablet number of the medicine searched manually in the tablet number input item 1520 and performs the click operation to the "OK" item 1530, the image processing program 600 references (displays) the medicine manually searched as the medicine search result together with the tablet number in the fourth part 740 (step S703). Fig. 49 is a drawing of one example for the condition in that the medicines manually searched are displayed on the judgement screen as the judgement result. As shown in Fig. 49, in the fourth part 740 of the "tablet image judgement" window 700, the medicine 1540 manually searched is displayed as the judgement object medicine together with the tablet number. According to the present embodiment, for example, even if the automatic judgment by the computer 500 is hard such as for a PTP sheet medicine because of absence of a barcode, the worker may add to the judgement list by searching manually so that usability may be improved.

### Section 3.12 Continuous Number Printing for WEB Judgement

Next, continuous number printing for WEB judgement will be explained. Fig. 50 is a flowchart of the continuous number printing work. Fig. 51,51 are one example of the printed medicine judgement result. Fig. 51 is one example of the printed medicine judgement result when addition of the barcode is set by a printed barcode goods identifier (AI code) addition setting 749 in the environment setting screen 744 of Fig. 21. On the other hand, Fig. 52 is a one example of the medicine judgement result when a barcode is not attached by a printed barcode goods identifier (AI code) addition setting 749 in the environment setting screen 744 of Fig. 21.

When the judgement for the medicine brought by the patient is completed and an operation for printing the judgement result from the worker is performed, the image processing program 600 receives a signal for directing the print of the judgement result (step S801). When the image processing program 600 receives the signal for directing the print, it prints the medicine judgement result on the medium (step S802) and outputs an identification number for identifying the result of the medicine judgement on the medium (step S803). Particularly, as shown in Fig. 51, in the medium a patient name 1610 and a judged person 1620 together with the barcode No 1630 and a barcode 1640, and the like are printed.

In the barcode No 1639, a machine No. of the apparatus that performed the judgement (one digit), a date of judgement (four digits) and a continuous number (3 digits). Since the continuous number is the number that is attached sereally every time the medicine judgement is done, the continuous number indicates numbers of the medicine judgement and plays a role of an ID number that identifies uniquely an individual medicine judgement (medicine judgement result). In addition, in the barcode No 1630, contents of the barcode No 1630 and contents of judgement result. With respect to the judged person, a name of a person logged in the system for the medicine judgement. According to the present embodiment, the judgement results may be retrieved easily in the WEB judgement only by scanning the barcode 1640 with a barcode reader.

On the other hand, in the printed barcode goods identifier (AI code) in the environment setting screen 744 of Fig. 21, when the setting that the barcode is not attached is set, as shown in Fig. 52, a dish No 1650, usage 1652, and a judgement medicine list 1654 are printed as well as the patient name 1610 and the judged person. In the dish No. 1650, the number of the dish used when the judgement is performed is printed; in the usage 1652, for example, the usage of the medicines set in Fig. 38-Fig. 40 is printed. The medium without the barcode as shown in Fig. 52, for example, the judgement result will be displayed intelligibly by pasting to a powder wrapping paper after the medicine judgement is finished. According to the present embodiment, depending on a purpose of work, two kinds of the judgement results may be printed so that an efficiency of the judgement work by the worker may be improved.

### Section 3.13 Region Editing of Medicine

Next, a region editing function of the medicine will be explained. Fig. 53 is a drawing for explaining the region editing function. Hereinbelow, in the case that the medicines brought by the patient is judged and after the completion of all of the medicines brought, one example for performing the region editing of the medicine will be explained, but not limited thereto. For example, the region edition may also be performed when the returned medicines are judged and even when the judgement of all of the medicines is not completed, the region edition of the medicine may be performed.

When the medicines are judged, the "tablet image judgement" window 700 shown in Fig. 53 is displayed on the display device 530. Fig. 53 provides as for convenience of explanation, a part of the "tablet image judgement" window 700 as an enlarged display. As shown in Fig. 53, in the "tablet image judgement" window 700, the region editing item 1700 is displayed.

When the worker performs the click operation to the region editing item 1700, the region editing screen 1710 shown in Fig. 54 is displayed on the display device 530. Fig. 54 is a drawing of one example of the region editing screen. As shown in Fig. 54, in the region editing screen 1710, the image 1720 that has been taken by the medicine photographing device 200, a "determination" item 1730, and a "close" item are displayed. As shown in Fig. 54, the computer 500 (image processing program 600), when judging the medicines based on the images photographed by the medicine photographing device 200, recognizes automatically the region that the medicine occupies. Now, Fig. 54 illustrates one example in that the medicine 1722 that was not recognized automatically by the computer 500 is present.

When the "determination" item 1730 is selected by the click operation by the worker, the result edited by the cutting-off region editing described below is fixed. In addition, when the "close" item is selected by the click operation by the worker, the region editing screen 1710 is closed and returns to the "tablet image judgement" window 700 again.

When the worker performs the click operation to any of the medicines in the image 1720, the cutting-out region editing screen 1740 shown in Fig. 55 is displayed on the display device 530. Fig. 55 is a drawing of one example of the cut-out region editing screen. As shown in Fig. 55, in the cutting-out region editing screen 1740, an "automatic rotation" item 1741, an "automatic rotation" item 1742, an "OK" item 1743, a "reset" item 1744, an "enlarge" item 1745, a "scale down" item 1746, a "move" item 1747, a "rotation" item 1748 and a "cancel" item 1749 are displayed.

The "automatic rotation" item 1741 is a button for rotating a display angle of a top image that the medicine is photographed from the above. The "automatic rotation" item 1742 a button for rotating a display angle of a bottom image that the medicine is photographed from the below. When the "automatic rotation" item 1741 or the "automatic rotation" item 1742 is selected by the click operation by the worker, the display angle of the top image or the bottom image is rotated so that the display angle of the top image and the bottom image is matched each other. When the "OK" item 1743 is selected by the click operation by the worker, the region editing is terminated to return to the region editing screen 1710.

When the "reset" item 1744 is selected with the click operation by the worker, the edited contents are cleared. When the "enlarge" item 1745 is selected with the click operation by the worker, the top image and the bottom image are enlarged. On the other hand, the "scale down" item 1746 is selected with the click operation by the worker, the top image and the bottom image are scaled down. As indicated by the "move" item 1747, when the worker drags under the condition that it keeps the mouse at left click operation, a position of the top image and/or the bottom image is moved. Furthermore, as indicated by the "rotation" item 1748, when the worker drags under the condition that it keeps the mouse at right click operation, the top image and/or the bottom image rotate by amounts of drag with respect to the direction of the drag. When the "cancel" item 1749 is selected with the click operation by the worker, the cut-off region editing screen 1740 is closed to return to the region editing screen 1710.

The worker may correct and add the region through each item and the mouse operation in the cut-out region editing screen 1740 with respect to the medicines that the automatic recognition is failed by the computer and the medicines that the automatic recognition is succeeded but correct regions are not recognized. Fig. 56 is a schematic illustration of one example of the region editing for the medicine. For example, as shown in Fig. 56 (1), when the region 1752 of the medicine 1750 is not recognized correctly by the computer 500, the worker selects the medicine of which region in the image 1720 is not recognized correctly by the click operation. Next, the worker in the cut-off region editing screen 1740 adjusts positions of the top image and/or the bottom image, sizes, or angles adequately and by selecting the "OK" item 1743 by the click operation, the region 1754 of the medicine 1750 may be corrected. In addition, as shown in Fig. 56 (2), when the region of the medicine 1750 is not recognized at all by the computer 500, the worker selects the medicine of which region is not recognized at all in the image 1720 by the click operation. Next, the worker in the cut-out region editing screen 1740 adjusts positions of the top image and/or the bottom image, sizes, or angles adequately and by selecting the "OK" item 1743 by the click operation, the region 1756 of the medicine 1750 may be newly added. When the region 1756 is newly added, the worker may search the judgment object medicine manually using the "manual search" item 1400 shown in Fig. 46.

### Section 4 Medicine Folding System

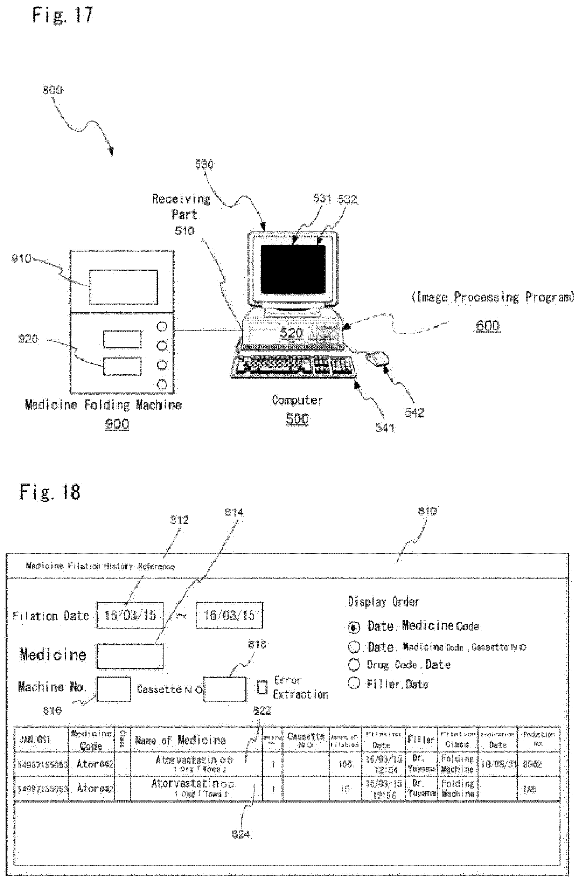

Next, a medicine folding system will be explained. Fig. 17 is a schematic drawing illustrating an outline of the medicine folding system of the present invention. As shown in the figure, the medicine folding system 800 comprises a medicine folding machine 900 and a computer 500. The medicine folding machine 900 comprises a display device 910 as an interface for outputting various information about the folding of the medicine and a barcode reader 920 for reading the barcode. The medicine folding machine 900 is connected to the computer 500. A receiver part 510 of the computer 500 receives, for example, various information such as histories of the medicines folded by the medicine folding apparatus 900 from the medicine folding machine 900. Hereupon, as convenience of explanation, with respect to the computer connected to the medicine folding machine 900 is the similar one with the computer 500, but not limited thereto, the other computer may be used.

The workers using the barcode printed on the judgement result shown in Fig. 15 fill the returned medicines to the medicine folding machine 900. That is to say, the worker first scans the barcode printed on the judgement result with the barcode reader 920. Then the worker scans the barcode printed on the medicine cassette to which the returned medicines are filled by the barcode reader 920. Thereby, the medicine folding machine 900 checks whether or not a kind of the returned medicine and a kind of to the medicine cassette correspond each other. Particularly, the barcode of the object medicine is read with the barcode reader 920. The medicine folding machine 900 is disposed with a plurality of medicine cassette to be the object of filling up and the medicine cassette for an object of filing is moved to the front of the machine body for easy pick up of worker and LED etc. of the object cassette is turned on and the like to promote not making mistakes for the removal. Furthermore, the medicine cassette is disposed with RFID to which the medicine information is written and when the cassette is removed from the body and is placed on a cassette information reader part (not shown) of the medicine folding machine 900, the medicine information is read from RFID to check whether or not the information matches with the medicine information of the barcode. As the result of check, when the determination that both matches each other, the worker may charge the returned medicine to the medicine cassette.

### Section 4.1 Traceability of Filled Medicine

Next, traceability of the returned medicine filled into the folding machine will be explained. Fig. 18 is a drawing of one example for an inquiry screen of a filling history of the medicine filled in the folding machine. As shown in Fig. 18, the image processing program 600, when a request for inquiring the history of the filled medicines, is received from the user, displays a screen (medicine filling history inquiry screen 810) for inquiring the history of the medicines filled into the folding machine on the display device 530. The user of the folding machine 900 in the medicine filling history inquiry screen 810 may inquire the filling history of the medicines into the folding machine by inputting at least one of a filling date 812, a medicine name 814, a machine number of the folding machine 816 and/or a medicine cassette No 818 as a search key. In Fig. 18, an example, in that by inputting the filling date as the search key, two cases for the filling of the medicine are searched, is illustrated. Here, the medicine filling history inquiry screen 810 may be displayed on the display device 910 which is disposed to the folding machine 900.

The medicine 822 in Fig. 18 is one that new medicines are filled into the folding machine rather than the returned medicines. The user of the folding machine may refer about the medicine 822 a medicine code, a medicine name, filling amount, a filling date, a filled person, an expiration date and production number, and the like. On the other hand, the medicine 824 is one that the returned and judged medicines are filled into the folding machine. As shown in Fig. 18, with respect to the returned and judged medicines, the user of the folding machine may refer the medicine code, the medicine name, the filling amount, the filling date and the filled person. Furthermore, with respect to the medicine 824, the letters "TAB" is displayed in a field of the production number. The letters "TAB" means that the returned medicines were filled. By displaying such letters and by referring the medicine filling history inquiry screen 810, the user of the folding machine may recognize that the medicine 824 is the returned and judged medicine.

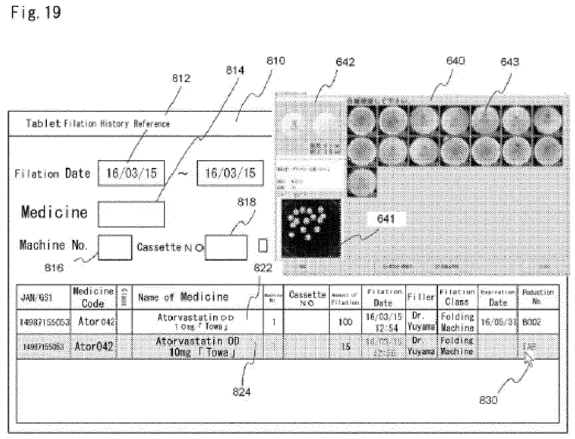

In addition, the image processing program 600 with correlating to the returned and judged medicine in the medicine filling history inquiry screen 810 may display at least one of the photographed images displayed on the first part 641 and the sorted images of a plurality of medicines displayed on the third part 643. That is to say, as shown in Fig. 19, when the user of the folding machine selects with moving the mouse cursor 830 to the row of the medicine 824, the row of the medicine 824 is highlighted. Here, corresponding to the selection of the medicine 824 in the medicine filling history inquiry screen 810, the "returned tablet image judgement" window 640 at the time when the return and judgement of the medicine 824 have performed. The user of the folding machine, by referring to the "returned tablet image judgement" window 640 in the medicine filling history inquiry screen 810, may trace which of the returned medicines was filled into the folding machine. For example, if filling of an inadequate medicine into the folding machine is found, which step the medicine was filled may be traced by referring the "returned tablet image judgement" window 640.

Now, in the example of Fig. 19, the example in that the "returned tablet image judgement" window 640 is displayed, but not limited thereto. The image processing program 600 may display at least one of the photographed images displayed on the first part 641 and the sorted images of the plurality of images displayed on the third part 643.

### Section 5 Referenced Invention

Hereinafter, the referenced invention will be acknowledged as an appendix.

### (Reference Invention 1)

An image processing apparatus comprising:
a receiver part for receiving an image taking a picture of a region including plurality of medicines by a medicine photographing apparatus and
an image processing part for executing the steps of; displaying an image photographing a region including plurality of medicines on a display device; and displaying the images of the plurality of medicines on the display device with sorting depending on a similarity degree between an image of a representative medicine selected from the plurality of medicines displayed on the display device and each of the images of the plurality of medicines.

### (Reference Invention 2)

The image processing apparatus of the reference invention 1, further making the computer execute the step of displaying on the display device an enlarged image of the representative image within an image of the photographed region or a master image corresponding to the representative medicine.

### (Reference Invention 3)

The apparatus of the reference invention 2, wherein the image taking a picture of the region is displayed in a first part of the display device; the enlarged image or the master image is displayed on a second part of the display device and the images with sorting the images of the plurality of medicines are displayed on a third part of the display device.

### (Reference Invention 4)

The apparatus of any one of the referenced inventions of 1-3, wherein the image processing part displays with sorting the images of the plurality of medicines displays the images of the plurality of medicines with sorting in a descending order or in an ascending order of the similarity degree.

### (Reference Invention 5)

The apparatus of any one of the referenced inventions of 1-4 further comprises a printer part for printing results of the judgement of the plurality of medicines on a medium,
wherein the printer part prints on the medium an identifier indicating the plurality of medicines being medicines returned and judged.

### (Reference Invention 6)

The image processing apparatus of any one of the referenced inventions 5, wherein the printer part prints with incorporating an identifier indicating the plurality of medicines being medicines returned and judged in a part of a barcode indicating a rot number printed on the medium.

### (Reference Invention 7)

The image processing apparatus of any one of the referenced inventions of 1-6, wherein the image processing part displays that the plurality of medicines are medicines returned and judged on a display device when displaying a history image indicating a history of a medicine filled into a folding machine.

### (Reference Invention 8)

The image processing apparatus of the referenced invention 7, wherein the image processing part displays on the display device at least one of the images of the photographed region and the images with sorting the plurality of medicines with correlating to a returned and judged medicine in the history image.

### (Reference Invention 9)

The image processing apparatus of the reference invention 5, wherein the printer part prints a tablet number of the plurality of medicines on the medium.

### (Reference Invention 10)

The image processing apparatus of the reference invention 5, wherein the printer part prints on the medium a total medicine price of the plurality of medicines judged based on a tablet number of a plurality of tablets judged and each of medicine prices of the plurality of medicines.

### (Reference Invention 11)

The image processing apparatus of any one of the reference inventions of 1-10, wherein the image processing part measures judgement time required for judging the plurality of medicines and accumulating the measured judgement time or the judgment time per tablet for the plurality of medicines.

### (Reference Invention 12)

The image processing part of any one of the referenced inventions of 1-11, wherein the image processing apparatus measures judgement time required for judging the plurality of medicines and accumulating the measured judgement time and the judgment time per tablet for the plurality of medicines.

### (Reference Invention 13)

The image processing apparatus of any one of the reference inventions of 1-12, wherein the image processing apparatus displays on the display device information of a candidate for a judgement of medicines, wherein the step of displaying on the display device the information of the candidate for a judgement of medicines, when a message item is included in the candidate medicines, displays on the display device an identifier for noticing presence of the massage item and displays the message item on the display device when the candidate medicine is selected.

### (Reference Invention 14)

The image processing apparatus of any one of the reference inventions of 1-13, wherein the image processing part, in a case that medicines brought by a patent are judged, displays on the display device a judgement screen for judging brought medicines of the patient; receives through the judgement screen displayed on the display device input of information about the patient brought the medicines and correlates the information about a patient brought the medicines to a result of judgement of the brought medicines.

### (Reference Invention 15)

The image processing apparatus of any one of the reference inventions of 1-14, wherein the image processing part, in a case that medicines brought by a patent are judged, displays on the display device a judgement screen for judging brought medicines of the patient; receives through the judgement screen displayed on the display device input of information about usage of the patient brought the medicines and correlating the information about the usage of the brought medicines by the patient to a result of judgement of the brought medicines.

### (Reference Invention 16)

The image processing apparatus of any one of the reference inventions of 1-15, wherein the image processing part displays a menu screen on the display device about a judgement of medicines and receives through the menu screen input for a change in a setting item about a judgement of the medicines.

### (Reference Invention 17)

The image processing apparatus of any one of the reference inventions of 16, wherein the setting item comprise usage of medicines to be objectives of the judgement.

### (Reference Invention 18)

The image processing apparatus of any one of the reference inventions of 1-17, wherein the image processing part displays a judgement screen for judging medicines on the display device; displays through a judgement screen for judging the medicines a screen for searching the medicines manually on the display device, and displays information of medicines input through a screen for searching the medicines manually as a result of a judgement of the medicines on the display device

### (Reference Invention 19)

The image processing apparatus of any one of the reference inventions of 1-18, wherein the image processing part prints a result of a judgement of medicines on a medium, wherein the printing step prints an identifying number for identifying a result of a judgement of the medicines as well as a result of a judgement of the medicine on the medium.

### (Reference Invention 20)

The image processing apparatus of any one of the reference inventions of 1-19, wherein the image processing part recognizes a region occupied by a medicine to be a judgement object and corrects a region recognized by the recognition step in response to an operation performed by a worker.

### (Reference Invention 21)

The image processing apparatus of any one of the reference inventions of 1-20, wherein the image processing part recognizes a region occupied by a medicine to be a judgement object and adds a region being not recognized by the recognition step in response to an operation performed by a worker.

### DESCRIPTION OF SIGNS

100---medicine judgement system
200---medicine photographing device
500---computer
510---receiver part
520---main body
530---display device
532---screen
532---touch screen
541---keyboard
542---mouse
600---image processing program
640---"returned tablet image judgement" window
641---first part
642---second part
643---third part
644---count and message display part
738---total number of returned medicines
742---total medicine price
782---identifier
784---message item
800---medicine folding system
900---medicine folding apparatus
1100---fifth part
1200---sixth part
1300---setting change screen
1400---"manual search" item
1630---barcode No
1700---region editing item

## Claims

1. A program making a computer execute the steps of:
displaying on a display device an image taking a picture of a region including a plurality of medicines, and
displaying the images of the plurality of medicines on the display device with sorting depending on a similarity degree between an image of a representative medicine selected from the plurality of medicines displayed on the display device and each of the images of the plurality of medicines.

2. The program of claim 1, further making the computer execute the step of:
displaying on the display device an enlarged image of the representative image within an image of the photographed region or a master image corresponding to the representative medicine.

3. The program of claim 2, wherein the image taking a picture of the region is displayed in a first part of the display device, the enlarged image or the master image is displayed on a second part of the display device, and
the images with sorting the images of the plurality of medicines are displayed on a third part of the display device.

4. The program of any one of claims 1-3, wherein the step for displaying the display device with sorting the images of the plurality of medicines displays the images of the plurality of medicines with sorting in a descending order or in an ascending order of the similarity degree.

5. The program of any one of claims 1-4, wherein the step for displaying on the display device with sorting the images of the plurality of medicines displays the images of the plurality of medicines with aligning directions of the images of the plurality of medicines with referencing the image of the representative medicine.

6. The program of any one of claims 1-5, wherein the step for displaying on the display device with sorting the images of the plurality of medicines displays the images of the plurality of medicines with aligning front and back of the plurality of medicines with referencing the front and back of the representative medicine in the image of the representative medicine.

7. The program of any one of claims 1-6, further making the computer execute the step of:
displaying information of medicines to be candidates for judging the representative medicine.

8. The program of any one of claims 1-7, further making the computer execute the step of:
printing results of the judgement of the plurality of medicines on a medium,
wherein the step of printing prints on the medium an identifier indicating the plurality of medicines being medicines returned and judged.

9. The program of claim 8, wherein the step of printing prints with incorporating an identifier indicating the plurality of medicines being medicines returned and judged in a part of a barcode indicating a rot number printed on the medium.

10. The program of any one of claims 1-8, further making the computer execute the step of:
displaying on the display device a history image indicating a history of a medicine filled into a folding machine,
wherein the step of displaying on the display device the history image displays that the plurality of medicines are medicines returned and judged.

11. The program of claim 10, further making the computer execute the step of:
displaying on the display device at least one of the images of the photographed region and the images with sorting the plurality of medicines with correlating to a returned and judged medicine in the history image.

12. The program of claim 8, wherein the step of printing prints a tablet number of the plurality of medicines judged on the medium.

13. The program of claim 8, wherein the step of printing prints on the medium a total medicine price of the plurality of medicines judged based on a tablet number of a plurality of tablets judged and each of medicine prices of the plurality of medicines.

14. The program of any one of claims 1-13, further making the computer execute the step of:
measuring judgement time required for judging the plurality of medicines and accumulating the measured judgement time or the judgment time per tablet for the plurality of medicines.

15. The program of claims 1-13, further making the computer execute the step of:
measuring judgement time required for judging the plurality of medicines and accumulating the measured judgement time and the judgment time per tablet for the plurality of medicines.

16. The program of any one of claims 1-15, further making the computer execute the step of:
displaying on the display device information of a candidate for a judgement of medicines,
wherein the step of displaying on the display device the information of the candidate for a judgement of medicines, when a message item is included in the candidate medicines, displays on the display device an identifier for noticing presence of the massage item and displays the message item on the display device when the candidate medicine is selected.

17. The program of any one of claims 1-16, further making the computer execute the steps of:
in a case that medicines brought by a patent are judged, displaying on the display device a judgement screen for judging brought medicines of the patient,
receiving through the judgement screen displayed on the display device input of information about the patient brought the medicines and
correlating the information about a patient brought the medicines to a result of judgement of the brought medicines.

18. The program of any one of claims 1-17, further making the computer execute the steps of:
in a case that medicines brought by a patent are judged, displaying on the display device a judgement screen for judging brought medicines of the patient,
receiving through the judgement screen displayed on the display device input of information about usage of the patient brought the medicines and
correlating the information about the usage of the brought medicines by the patient to a result of judgement of the brought medicines.

19. The program of any one of claims 1-18, further making the computer execute the steps of:
displaying a menu screen on the display device about a judgement of medicines, and
receiving through the menu screen input for a change in a setting item about a judgement of the medicines.

20. The program of claim 19, wherein the setting item comprise usage of medicines to be an objective for the judgement.

21. The program of any one of claims 1-20, making the computer execute the steps of:
displaying a judgement screen for judging medicines on the display device,
displaying through a judgement screen for judging the medicines a screen for searching the medicines manually on the display device,
displaying information of medicines input through a screen for searching the medicines manually as a result of a judgement of the medicines on the display device.

22. The program of any one of claim 1-21, further making the computer execute the step of:
printing a result of a judgement of medicines on a medium,
wherein the printing step prints an identifying number for identifying a result of a judgement of the medicines as well as a result of a judgement of the medicine on the medium.

23. The program of any one of claim 1-22, further making the computer execute the step of:
recognizing a region occupied by a medicine to be a judgement object,
correcting a region recognized by the recognition step in response to an operation performed by a worker.

24. The program of any one of claims 1-23, further making the computer execute the steps of:
recognizing a region occupied by a medicine to be a judgement object,
adding a region being not recognized by the recognition step in response to an operation performed by a worker.

25. An image processing apparatus comprising:
a receiver part for receiving an image taking a picture of a region including plurality of medicines by a medicine photographing apparatus,
an image processing part for executing displaying an image photographing a region including plurality of medicines on a display device, and displaying the images of the plurality of medicines on the display device with sorting depending on a similarity degree between an image of a representative medicine selected from the plurality of medicines displayed on the display device and each of the images of the plurality of medicines.
